# FASCICULE DE BREVET EUROPEEN

(11) **EP 0 817 780 B1**
(45) Date de publication et mention de la délivrance du brevet: **13.10.1999**
(21) Numéro de dépôt: 96909193.3
(22) Date de dépôt: 25.03.1996
(51) Int. Cl.: C07D 305/14, A61K 31/335

(54) **NOUVEAUX TAXO DES, LEUR PREPARATION ET LES COMPOSITIONS PHARMACEUTIQUES QUI LES CONTIENNENT**
TAXOL DERIVATE, DEREN HERSTELLUNG UND DIESE ENTHALTENDE PHARMAZEUTISCHEN ZUSAMMENSTELLUNGEN
NOVEL TAXOIDS, PREPARATION THEREOF AND PHARMACEUTICAL COMPOSITIONS CONTAINING SAME

(30) Priorité: 27.03.1995 FR 9503545; 22.12.1995 FR 9515381
(43) Date de publication de la demande: 14.01.1998
(73) Titulaire: RHONE-POULENC RORER S.A., 92160 Antony (FR)
(72) Inventeur: BOUCHARD, Hervé, F-94320 Thiais (FR); BOURZAT, Jean-Dominique, F-94300 Vincennes (FR); COMMERCON, Alain, F-94400 Vitry-sur-Seine (FR)
(74) Mandataire: Le Pennec, Magali
(86) Numéro de dépôt international: FR9600441
(87) Numéro de publication internationale: WO9630356

(56) Documents cités:
- EP-A- 0 639 577
- WO-A-94/18164

## Description

La présente invention concerne de nouveaux taxoïdes de formule générale : 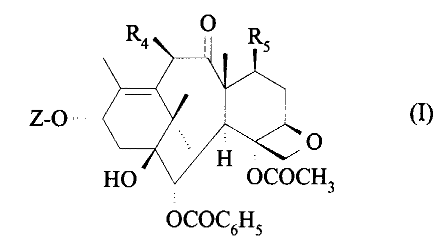 dans laquelle
Z représente un atome d'hydrogène ou un radical de formule générale : 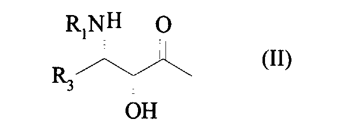 dans laquelle :
R₁ représente
   - un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle
   - ou un radical R₂-O-CO- dans lequel R₂ représente :
      - un radical alcoyle contenant 1 à 8 atomes de carbone, alcényle contenant 2 à 8 atomes de carbone, alcynyle contenant 3 à 8 atomes de carbone, ²cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
      - un radical phényle ou *α-* ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
      - ou un radical hétérocyclyle saturé contenant 4 à 6 atomes de carbone éventuellement substitué par un ou plusieurs radicaux alcoyles contenant 1 à 4 atomes de carbone,
R₃ représente :
   - un radical alcoyle droit ou ramifié contenant 1 à 8 atomes de carbone,
   - alcényle droit ou ramifié contenant 2 à 8 atomes de carbone,
   - alcynyle droit ou ramifié contenant 2 à 8 atomes de carbone,
   - cycloalcoyle contenant 3 à 6 atomes de carbone,
   - phényle ou *α-* ou β-naphtyle
   éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro et trifluorométhyle,
   - ou un hétérocycle aromatique ayant 5 chaînons et contenant un ou plusieurs hétéroatomes, identiques ou différents, choisis parmi les atomes d'azote, d'oxygène ou de soufre et
   éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
R₄ représente :
   - un radical alcanoyloxy dont la partie alcanoyle contient 2 à 6 atomes de carbone en chaîne droite ou ramifiée, à l'exception du radical acétyle,
   - alcénoyloxy dont la partie alcénoyle contient 3 à 6 atomes de carbone en chaîne droite ou ramifiée,
   - alcynoyloxy dont la partie alcynoyle contient 3 à 6 atomes de carbone en chaîne droite ou ramifiée,
   ces radicaux étant éventuellement substitués par un ou plusieurs atomes d'halogène ou par un radical alcoxy contenant 1 à 4 atomes de carbone, alcoylthio contenant 1 à 4 atomes de carbone, ou par un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical cycloalcanoyloxy dont la partie cycloalcanoyle contient 4 à 8 atomes de carbone ou un radical cycloalcénoyloxy dont la partie cyccloalcénoyle contient 4 à 8 atomes de carbone, ou bien R₄ représente un radical benzoyloxy ou hétérocyclylcarbonyloxy dans lequel la partie hétérocyclique représente un hétérocycle aromatique 5 ou 6 chaînons contenant un ou plusieurs hétéroatomes choisis parmi les atomes d'oxygène, de soufre ou d'azote,
R₅ représente :
   - un radical alcoxy contenant 1 à 6 atomes de carbone en chaîne droite ou ramifiée éventuellement substitué par un radical alcoxy contenant 1 à 4 atomes de carbone,
   - alcényloxy contenant 3 à 6 atomes de carbone,
   - alcynyloxy contenant 3 à 6 atomes de carbone,
   - cycloalcoyloxy contenant 3 à 6 atomes de carbone,
   - cycloalcényloxy contenant 3 à 6 atomes de carbone,
   ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone.

De préférence les radicaux aryles pouvant être représentés par R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluoro-méthyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R₃ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

De préférence le radical R₄ représente un radical alcoyloxyacétoxy dont la partie alcoyle contient 1 à 4 atomes de carbone, cycloalcanoyloxy dont la partie cycloalcanoyle contient 4 à 8 atomes de carbone, cycloacénoyloxy dont la partie cycloalcénoyle contient 4 à 8 atomes de carbone, benzoyloxy ou hétérocyclylcarbonyloxy dans lequel la partie hétérocyclique représente un hétérocycle aromatique 5 ou 6 chaînons contenant un ou plusieurs hétéroatomes choisis parmi les atomes d'oxygène, de soufre ou d'azote, et R₅ représente un radical alcoxy droit ou ramifié contenant 1 à 6 atomes de carbone éventuellement substitués par un radical méthoxy, éthoxy, méthylthio, éthylthio, carboxy, méthoxycarbonyle, éthoxycarbonyle, cyano, carbamoyle, N-méthyl-carbamoyle, N-éthylcarbamoyle, N,N-diméthyl-carbamoyle, N,N-diéthylcarbamoyle, N-pyrrolidinocarbonyle ou N-pipéridino-carbonyle.

Plus particulièrement, la présente invention concerne les produits de formule générale (I) dans laquelle
Z représente un atome d'hydrogène ou un radical de formule générale (II) dans laquelle R₁ représente un radical benzoyle ou un radical R₂-O-CO- dans lequel R₂ représente un radical tert-butyle
et R₃ représente:
   - un radical alcoyle contenant 1 à 6 atomes de carbone,
   - alcényle contenant 2 à 6 atomes de carbone,
   - cycloalcoyle contenant 3 à 6 atomes de carbone,
   - phényle
   éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle
   - ou un radical furyle-2 ou -3,
   - thiényle-2 ou -3 ou
   - thiazolyle-2, -4 ou -5 et
R₄ représente un radical alcoyloxyacétoxy dont la partie alcoyle contient 1 à 4 atomes de carbone, cycloalcanoyloxy dont la partie cycloalcanoyle contient 4 à 6 atomes de carbone, pyridylcarbonyloxy et R₅ représente un radical alcoyloxy droit ou ramifié contenant 1 à 6 atomes de carbone.

Plus particulièrement encore, la présente invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ représente un radical méthoxyacétoxy, cyclopropylcarbonyloxy, cyclopentylcarbonyloxy, pyridyl-2 carbonyloxy ou pyridyl-3 carbonyloxy et R₅ représente un radical méthoxy.

Il était connu selon la demande de brevet WO 94/18164 un procédé de préparation de taxoïdes par condensation d'un motif β-lactam avec un noyau baccatine protégé en vue de préparer éventuellement le Taxotère.

Aucun moyen de préparer des noyaux taxoïdes porteurs d'un groupe alkoxy en position 7 n'est décrit.

Il est également connu selon la demande de brevet EP 639577 une série de dérivés antitumoraux constitués de dérivés de la classe des taxanes porteurs en position 7,10 ou 2' d'un motif éther de phosphonooxyméthyl ou de méthylthiométhyl. Rien ne permet à la lecture de ce document d'envisager de remplacer 1 motif spécifique tel que décrit ci-dessus par 1 motif alkoxy en position 7.

Les produits de formule générale (I) dans laquelle Z représente un radical de formule générale (II) présentent des propriétés antitumorales et antileucémiques remarquables.

Selon la présente invention, les nouveaux produits de formule générale (I) dans laquelle Z représente un radical de formule générale (II) peuvent être obtenus par estérification d'un produit de formule générale : 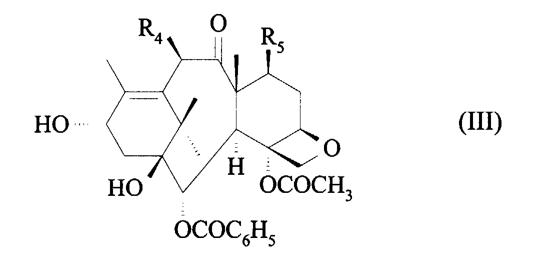 dans laquelle R₄ et R₅ sont définis comme précédemment, au moyen d'un acide de formule générale : 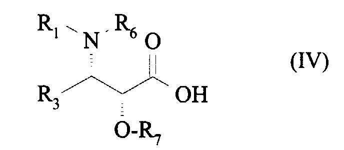 dans laquelle R₁ et R₃ sont définis comme précédemment, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 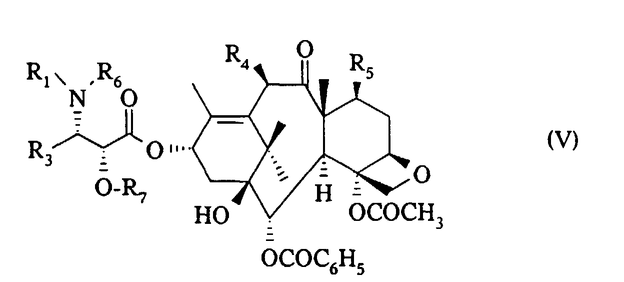 dans laquelle R₁, R₃, R₄, R₅, R₆ et R₇ sont définis comme précédemment, suivi du remplacement des groupements protecteurs représentés par R₇ et/ou R₆ et R₇ par des atomes d'hydrogène.

L'estérification au moyen d'un produit de formule générale (XII) dans laquelle X₁ représente un radical hydroxy peut être effectuée en présence d'un agent de condensation (carbodiimide, carbonate réactif) et d'un agent d'activation (aminopyridines) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant un produit de formule générale (XII) dans laquelle X₁ représente un radical R₄-O- en opérant en présence d'un agent d'activation (aminopyridines) dans un solvant organique (éthers, esters, cétones, nitriles, hydrocarbures aliphatiques, hydrocarbures aliphatiques halogénés, hydrocarbures aromatiques) à une température comprise entre 0 et 90°C.

L'estérification peut aussi être réalisée en utilisant un produit de formule géénrale (XII) dans laquelle X₁ représente un atome d'halogène, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

De préférence, R₆ représente un atome d'hydrogène et R₇ représente un groupement protecteur de la fonction hydroxy ou bien R₆ et R₇ forment ensemble un hétérocycle saturé à 5 ou 6 chaînons.

Lorsque R₆ représente un atome d'hydrogène, R₇ représente de préférence un radical méthoxyméthyle, éthoxy-1 éthyle, benzyloxyméthyle, triméthylsilyle, triéthylsilyle, β-triméthylsilyléthoxyméthyle, benzyloxycarbonyle ou tétrahydropyrannyle.

Lorsque R₆ et R₇ forment ensemble un hétérocycle, celui-ci est de préférence un cycle oxazolidine éventuellement mono-substitué ou gem-disubstitué en position -2.

Le remplacement des groupements protecteurs R₇ et/ou R₆ et R₇ par des atomes d'hydrogène peut être effectué, selon leur nature de la manière suivante :
1) lorsque R₆ représente un atome d'hydrogène et R₇ représente un groupement protecteur de la fonction hydroxy, le remplacement des groupements protecteurs par des atomes d'hydrogène s'effectue au moyen d'un acide minéral (acide chlorhydrique, acide sulfurique, acide fluorhydrique) ou organique (acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₆ et R₇ forment ensemble un hétérocycle saturé à 5 ou 6 chaînons et plus particulièrement un cycle oxazolidine de formule générale : 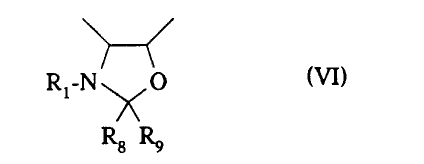 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, le remplacement du groupement protecteur formé par R₆ et R₇ par des atomes d'hydrogène peut être effectué, selon les significations de R₁, R₈ et R₉, de la manière suivante :
   a) lorsque R₁ représente un radical tert-butoxycarbonyle, R₈ et R₉, identiques ou différents, représentent un radical alcoyle ou un radical aralcoyle (benzyle) ou aryle (phényle), ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, le traitement de l'ester de formule générale (V) par un acide minéral ou organique éventuellement dans un solvant organique tel qu'un alcool conduit au produit de formule générale : 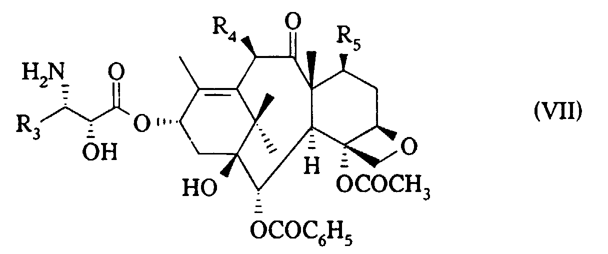 dans laquelle R₃, R₄ et R₅ sont définis comme précédemment, qui est acylé au moyen de chlorure de benzoyle dans lequel le noyau phényle est éventuellemnt substitué, de chlorure de thénoyle, de chlorure de furoyle ou d'un produit de formule générale :

      R₂-O-CO-X (VIII)

      dans laquelle R₂ est défini comme précédemment et X représente un atome d'halogène (fluor, chlore) ou un reste -O-R₂ ou -O-CO-O-R₂, pour obtenir un produit de formule générale (I) dans laquelle Z représente un radical de formule générale (II).
      De préférence, le produit de formule générale (V) est traité par l'acide formique à une température voisine de 20°C pour fournir le produit de formule générale (VII).
      De préférence, l'acylation du produit de formule générale (VII) au moyen d'un chlorure de benzoyle dans lequel le radical phényle est éventuellement substitué, de chlorure de thénoyle ou de chlorure de furoyle ou d'un produit de formule générale (VIII) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.
   b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, le remplacement du groupement protecteur formé par R₆ et R₇ par des atomes d'hydrogène s'effectue en présence d'un acide minéral (acide chlorhydrique, acide sulfurique) ou organique (acide acétique, acide méthanesulfonique, acide trifluorométhanesulfonique, acide p.toluènesulfonique) utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

Selon l'invention, les produits de formule générale (III), c'est-à-dire les produits de formule générale (I) dans laquelle Z représente un atome d'hydrogène, R₄ et R₅ sont définis comme précédemment, peuvent être obtenus à partir de la 10-désacétyl-baccatine III de formule :

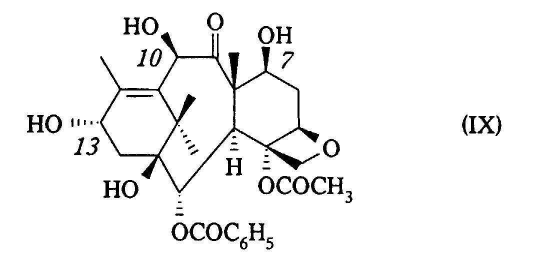

Il peut être particulièrement avantageux de protéger sélectivement les fonctions hydroxy en positions 7 et 13, par exemple sous forme d'un di-éther silylé qui peut être obtenu par action d'un halogénure de silyle de formule générale :

(R)₃-Si-Hal (X)

dans laquelle les symboles R, identiques ou différents, représentent un radical alcoyle contenant 1 à 4 atomes de carbone éventuellement substitué par un radical phényle, ou un radical phényle, sur la 10-désacétyl-baccatine III pour obtenir un produit de formule générale : 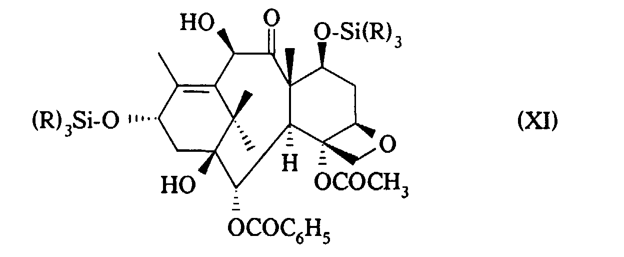 dans laquelle R est défini comme précédemment, puis action d'un produit de formule générale :

R'₄-X₁ (XII)

dans laquelle R'₄ est tel que R'₄-O- est identique à R₄ défini comme précédemment mais ne peut pas représenter un atome d'hydrogène ou un radical hydroxy et X₁ représente un atome d'halogène pour obtenir un produit de formule générale : 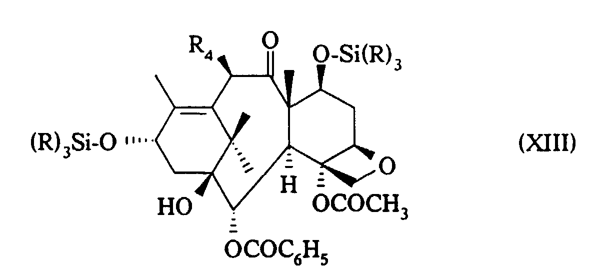 dans laquelle R et R₄ sont définis comme précédemment, dont les groupements protecteurs silylés sont remplacés par des atomes d'hydrogène pour obtenir un produit de formule générale : 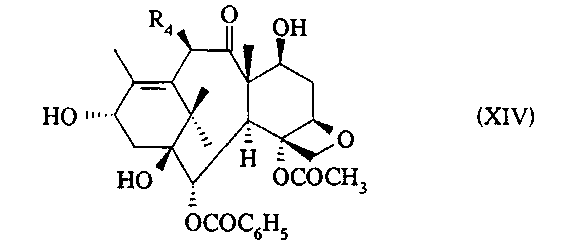 dans laquelle R₄ est défini comme précédemment, qui est éthérifié sélectivement en position 7 par action d'un produit de formule générale :

R'₅-X₂ (XV)

dans laquelle R'₅ est tel que R'₅-O- est identique à R₅ défini comme précédemment et X₂ représente atome d'halogène ou un reste d'ester sulfurique ou sulfonique pour donner le produit de formule générale (III).

Généralement, l'action d'un dérivé silylé de formule générale (X) sur la 10-désacétyl-baccatine III est effectuée dans la pyridine ou la triéthylamine éventuellement en présence d'un solvant organique tel qu'un hydrocarbure aromatique comme le benzène, le toluène ou les xylènes à une température comprise entre 0°C et la température de reflux du mélange réactionnel.

Généralement, l'action d'un produit de formule générale (XII) sur un produit de formule générale (XI), est effectuée, après métallation de la fonction hydroxy en position 10 au moyen d'un hydrure de métal alcalin tel que l'hydrure de sodium, un amidure de métal alcalin tel que l'amidure de lithium ou d'un alcoylure de métal alcalin tel que le butyllithium, en opérant dans un solvant organique tel que le diméthylformamide ou le tétrahydrofurane à une température comprise entre 0 et 50°C.

Généralement le remplacement des groupements protecteurs silylés du produit de formule générale (XIII) par des atomes d'hydrogène s'effectue au moyen d'un acide tel que l'acide fluorhydrique ou l'acide trifluoroacétique en présence d'une base telle que la triéthylamine ou la pyridine éventuellement substituée par un ou plusieurs radicaux alcoyles contenant 1 à 4 atomes de carbone, éventuellement associée à un solvant organique inerte tel qu'un nitrile comme l'acétonitrile ou un hydrocarbure aliphatique halogéné comme le dichlorométhane à une température comprise entre 0 et 80°C.

Généralement l'action d'un produit de formule générale (XV) sur un produit de formule générale (XIV) s'effectue dans les conditions indiquées précédemment pour l'action d'un produit de formule générale (XII) sur un produit de formule générale (XI).

Selon l'invention, les produits de formule générale (I) dans laquelle Z représente un radical de formule générale (II), R₄ et R₅ sont définis comme précédemment, peuvent être obtenus à partir d'un produit de formule générale : 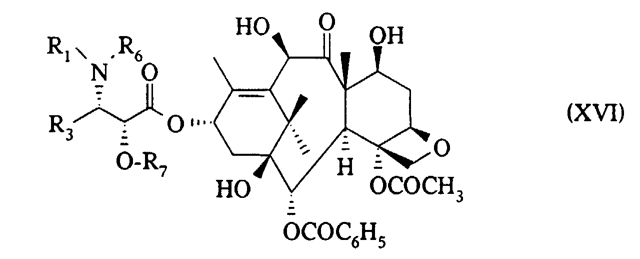 dans laquelle R₁, R₃, R₆ et R₇ sont définis comme précédemment par silylation en position 7 au moyen d'un produit de formule générale (X) pour obtenir un produit de formule générale : 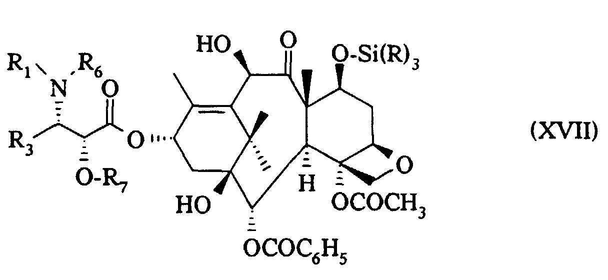 dans laquelle R, R₁, R₃, R₆ et R₇ sont définis comme précédemment, qui est fonctionnalisé en position 10 au moyen d'un produit de formule générale (XII) pour donner un produit de formule générale : 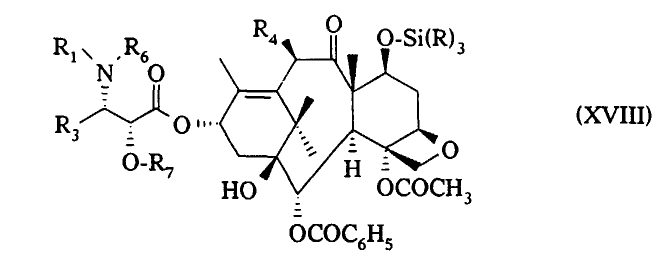 dans laquelle R, R₁, R₃, R₄, R₆ et R₇ sont définis comme précédemment dont le groupement protecteur silylé est remplacé par un atome d'hydrogène pour donner un produit de formule générale : 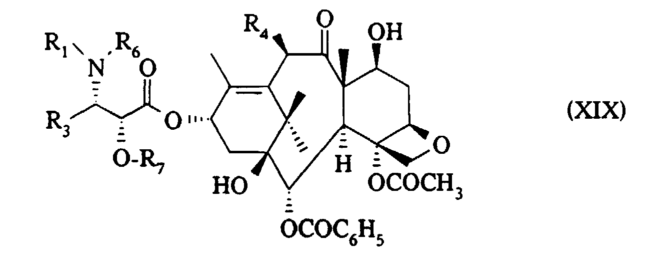 qui, par action d'un produit de formule générale (XV) conduit au produit de formule générale (V) dont les groupements protecteurs sont remplacés par des atomes d'hydrogène pour donner un produit de formule générale (I) dans laquelle Z représente un radical de formule générale (II).

Les réactions de silylation, de fonctionnalisation et de remplacement des groupements protecteurs par des atomes d'hydrogène sont effectuée dans des conditions analogues à celles décrites ci-dessus.

Les produits de formule générale (XVI) peuvent être obtenus dans les conditions décrites dans le brevet européen EP 0 336 841 et les demandes internationales PCT WO 92/09589 et WO 94/07878 ou à partir des produits de formule générale : 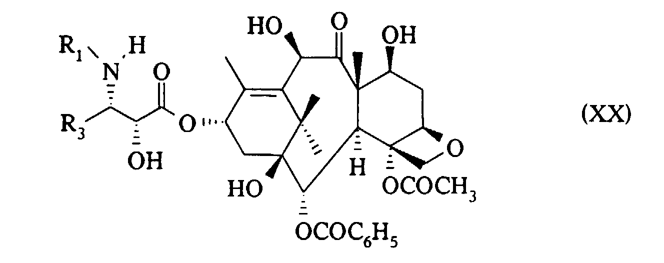 dans laquelle R₁ et R₃ sont définis comme précédemment selon les méthodes connues de protection de la fonction hydroxy de la chaîne latérale sans toucher au reste de la molécule.

Selon l'invention, les produits de formule générale (I) dans laquelle Z représente un atome d'hydrogène ou un radical de formule générale (II) peuvent être obtenus par action de nickel de Raney activé en présence d'un alcool aliphatique contenant 1 à 3 atomes de carbone sur un produit de formule générale : 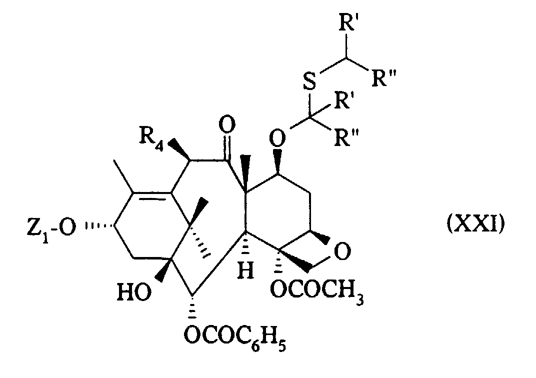 dans laquelle R₄ est défini comme précédemment et R' et R", identiques ou différents représentent un atome d'hydrogène ou un radical alcoyle contenant 1 à 6 atomes de carbone, alcényle contenant 2 à 6 atomes de carbone, alcynyle contenant 2 à 6 atomes de carbone, cycloalcoyle contenant 3 à 6 atomes de carbone ou cycloalcényle contenant 3 à 6 atomes de carbone, ou bien R' et R" forment ensemble avec l'atome de carbone auquel ils sont liés un radical cycloacoyle contenant 3 à 6 atomes de carbone ou un radical cycloalcényle contenant 4 à 6 atomes de carbone, et Z₁ représente un atome d'hydrogène ou un radical de formule générale : 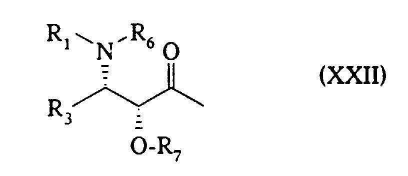 dans laquelle R₁ et R₃ sont définis comme précédemment et, ou bien, R₆ représente un atome d'hydrogène et R₇ représente un groupement protecteur de la fonction hydroxy, et, ou bien, R₆ et R₇ forment ensemble un hétérocycle saturé à 5 ou 6 chaînons, R₄ est défini comme précedemment, pour obtenir un produit de formule générale : 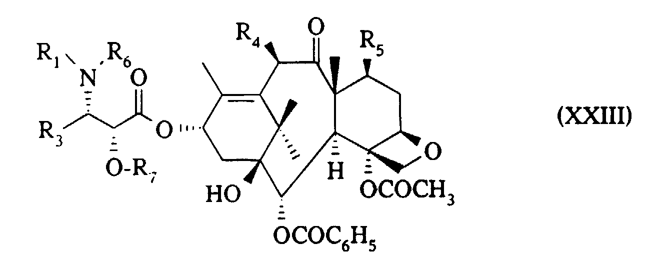 suivi, lorsque Z₁ représente un radical de formule générale (XXII), c'est-à-dire lorsque le produit de formule générale (XXIII) est identique au produit de formule générale (V), du remplacement des groupements protecteurs représentés par R₆ et/ou R₆ et R₇ par des atomes d'hydrogène dans les conditions décrites précédemment.

Généralement, l'action du nickel de Raney activé en présence de l'alcool aliphatique est effectuée à une température comprise entre -10 et 60°C.

Selon l'invention, le produit de formule générale (XXI) dans laquelle Z₁ et R₄ sont définis comme précédemment peut être obtenu par action d'un dialcoylsulfoxyde de formule générale: 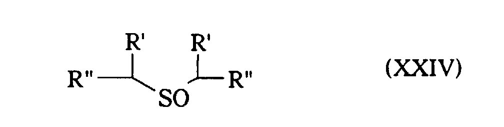 dans laquelle R' et R" sont définis comme précédemment, sur un produit de formule générale (XIX).

Généralement la réaction du sulfoxyde de formule générale (XXIV), de préférence le diméthylsulfoxyde, sur le produit de formule générale (XIX) s'effectue en présence d'un mélange d'acide acétique et d'anhydride acétique ou d'un dérivé de l'acide acétique tel qu'un acide halogénoacétique à une température comprise entre 0 et 50°C, de préférence voisine de 25°C.

Selon l'invention, les produits de formule générale (I) dans laquelle Z représente un radical de formule générale (II) peuvent être obtenus par action d'un produit de formule générale (XII) sur un produit de formule générale : 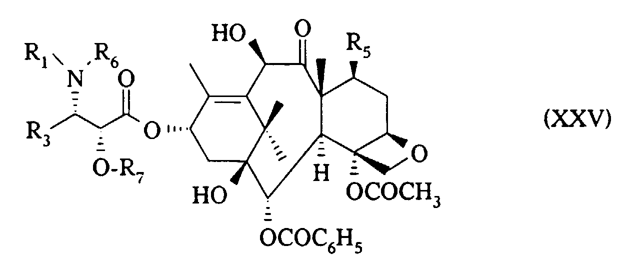 dans laquelle R₁, R₃, R₅, R₆ et R₇ sont définis comme précédemment, en opérant dans les conditions décrites pour l'action d'un produit de formule générale (XII) sur un produit de formule générale (XI), suivie du remplacement des groupements protecteurs représentés par R₇ ou R₆ et R₇ par des atomes d'hydrogène dans les conditions décrites précédemment.

Le produit de formule générale (XXV) peut être obtenu par action d'un halogénure de zinc tel que l'iodure de zinc ou l'hydrazine sur un produit de formule générale : 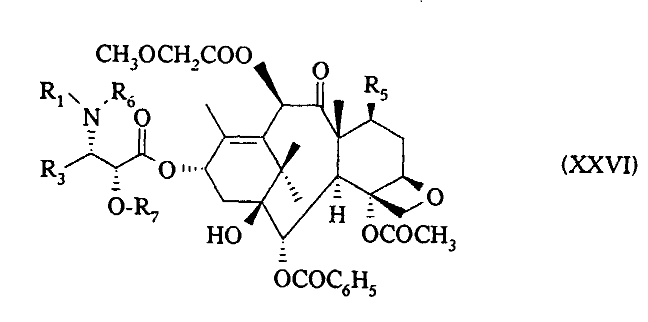 dans laquelle R₁, R₃, R₅, R₆ et R₇ sont définis comme précédemment.

Généralement la réaction est effectuée en opérant dans un alcool aliphatique contenant 1 à 4 atomes de carbone tel que le méthanol ou l'éthanol à une température comprise entre 0 et 50°C.

Le produit de formule générale (XXVI) peut être obtenu par action de nickel de Raney activé en présence d'un alcool aliphatique contenant 1 à 3 atomes de carbone sur un produit de formule générale : 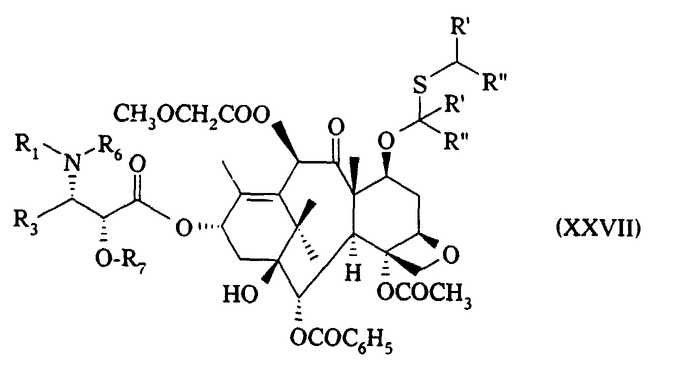 dans laquelle R₁, R₃, R₆, R₇, R' et R" sont définis comme précédemment, en opérant dans les conditions décrites précédemment pour la préparation d'un produit de formule générale (I) à partir d'un produit de formule générale (XXI).

Le produit de formule générale (XXVII) peut être obtenu par action d'un sulfoxyde de formule générale (XXIV) sur un produit de formule générale : 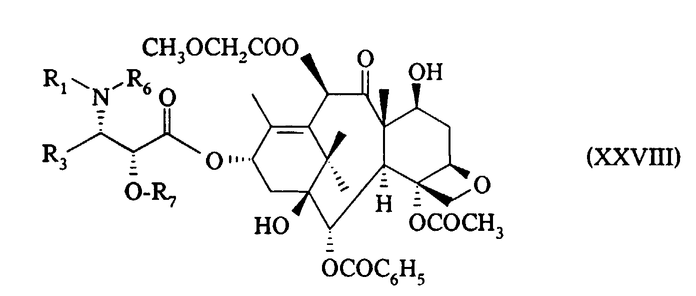 dans laquelle R₁, R₃, R₆ et R₇ sont définis comme précédemment, en opérant dans les conditions décrites précédemment pour l'action d'un sulfoxyde de formule générale (XXIV) sur un produit de formule générale (XIX).

Le produit de formule générale (XXVIII) peut être obtenu à partir d'un produit de formule générale : 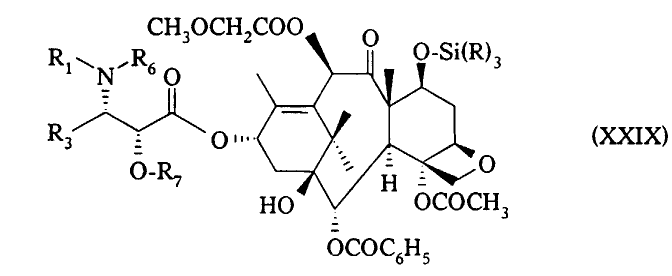 dans laquelle R₁, R₃, R₆ et R₇ sont définis comme précédemment, en opérant dans les conditions décrites précédemment pour remplacer les groupements sylilés du produit de formule générale (XIII) par des atomes d'hydrogène.

Le produit de formule générale (XXIX) peut être préparé dans les conditions décrites dans la demande internationale PCT WO 95/11241.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le taxol et le Taxotère.

In vivo, les produits de formule générale (I) dans laquelle Z représente un radical de formule générale (II) se sont montrés actifs chez la souris greffée par le mélanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug resistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX. une lignée cellulaire sélectionnée pour sa résistance à la doxorubicine (DOX) qui exprime mdr 1.

Les exemples suivants illustrent la présente invention.

### EXEMPLE 1

On dissout 243 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β oxo-9 (pyridyl-3 carbonyl)oxy-10β taxène-11 yle-13α dans 4,5 cm3 d'une solution éthanolique 0,1N d'acide chlorhydrique à 1% d'eau. La solution ainsi obtenue est agitée pendant 3 heures à une température voisine de 20°C puis additionnée de 25 cm3 de dichlorométhane. La phase organique est séparée par décantation et lavée successivement par 2 fois 10 cm3 d'une solution aqueuse saturée d'hydrogénocarbonate de sodium, séchée sur sulfate de magnésium, filtrée et concentrée à sec sous pression réduite (2,7 kPa) à 40°C. On obtient 290 mg d'une meringue blanche que l'on purifie par chromatographie sur gel de silice déposé sur plaques [(gel de 1mm d'épaisseur, plaques de 20 x 20 cm, éluant : dichlorométhane-méthanol (95-5 en volumes)] par fractions de 80 mg (4 plaques). Après localisation aux rayons U.V. de la zone correspondant au produit cherché adsorbé, cette zone est grattée et la silice recueillie est lavée sur verre fritté par 10 fois 10 cm3 d'acétate d'éthyle. Les filtrats sont réunis et concentrés à sec sous pression réduite (2,7 kPa) à 20°C. On obtient une meringue blanche que l'on repurifie selon la même technique [(2 plaques : 20 x 20 x 1 mm; éluant : dichlorométhane-méthanol (95-5 en volumes)]. On obtient ainsi 132 mg de tert-butoxycarbonylamino-3 hydroxy-2 phényl-3 propionate-(2R,3S) d'acétoxy-4α benzoyloxy-2α époxy-5β,20 hydroxy-1β méthoxy-7β oxo-9 (pyridyl-3 carbonyl)oxy-10β taxène-11 yle-13α sous forme d'une meringue blanche dont les caractéristiques sont les suivantes :
- pouvoir rotatoire : [α]^{D}₂₀ = - 34 (c = 0,5 ; méthanol).
- spectre de R.M.N. ¹H (300 MHz ; CDCl₃ ; déplacements chimiques δ en ppm ; constantes de couplage J en Hz) : 1,30 (s, 3H : -CH₃ en 16 ou en 17) ; 1,35 [(s, 12H : -C(CH₃)₃ et -CH₃ en 16 ou en 17] ; 1,75 (s, 3H : -CH₃) ; 1,82 et 2,77 (2 mts, 1H chacun : -CH₂- en 6) ; 1,97 (s, 3H : -CH₃) ; 2,35 (d, J = 9, 2H : -CH₂- en 14) ; 2,39 (s, 3H : -COCH₃) ; 3,38 (d, J = 5, 1H : -OH en 2'); 3,42 (s, 3H : -OCH₃) ; 3,88 (d, J = 7,5, 1H : -H en 3) ; 3,96 (dd, J = 11 et 7,5, 1H : -H en 7) ; 4,18 et 4,32 (2 d, J = 8,5, 1H chacun : -CH₂- en 20); 4,64 (mt, 1H : -H en 2'); 4,98 (d large, J = 10, 1H : -H en 5) ; 5,28 (d large, J = 10, 1H : -H en 3') ; 5,39 (d, J = 10, 1H : -CONH-) ; 5,70 (d, J = 7,5, 1H : -H en 2) ; 6,22 (t large, J = 9, 1H : -H en 13) ; 6,69 (s, 1H : -H en 10) ; de 7,25 à 7,45 (mt, 5H : -C₆H₅ en 3') ; 7,44 [(dd, J = 8,5 et 6, 1H : -OCOC₅H₄N(-H en 5)] ; 7,50 [(d, J = 7,5, 2H : -OCOC₆H₅(-H en 3 et H en 5)] ; 7,62 [(t, J = 7,5, 1H : -OCOC₆H₅(-H en 4)] ; 8,12 [(d, J = 7,5, 2H : -OCOC₆H₅ (-H en 2 et -H en 6)] ; 8,35 [(dt, J = 8,5 et 1, 1H : -OCOC₅H₄N(-H en 4)] ; 8,82 (dd, J = 6 et 1, 1H : -OCOC₅H₄N(-H en 6)] ; 9,32 (d, J = 1, 1H : -OCOC₅H₄N(-H en 2)].

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β oxo-9 (pyridyl-3 earbonyl)oxy-10β taxène-11 yle-13α peut être préparé de la manière suivante :

A une solution de 46 mg d'acide pyridine-3 carboxylique dans 25 cm3 d'acétate d'éthyle anhydre, maintenue sous atmosphère d'argon et sous agitation, on ajoute, à une température voisine de 20°C, 290 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10β méthoxy-7β oxo-9 taxène-11 yle-13α, 18,5 mg de diméthylamino-4 pyridine, 0,5 g de tamis moléculaire 4 Å et 112 mg de N,N'-dicyclohexylcarbodiimide. Le mélange réactionnel est maintenu sous agitation pendant 16 heures à une température voisine de 20°C puis on ajoute 46 mg d'acide pyridine-2 carboxylique, 18,5 mg de diméthylamino-4 pyridine, 0,5 g de tamis moléculaire 4 Å et 112 mg de N,N'-dicyclohexylcarbodiimide et maintient à nouveau sous agitation pendant 24 heures puis répète ce cycle deux autres fois. Le mélange réactionnel est filtré sur verre fritté garni de célite. Le verre fritté est lavé par 2 fois 50 cm3 d'acétate d'éthyle, les filtrats sont réunis, lavés successivement par 2 fois 10 cm3 d'une solution aqueuse saturée d'hydrogénocarbonate de sodium et par 6 fois 20 cm3 d'eau distillée, séchés sur sulfate de magnésium, filtrés et concentrés à sec sous pression réduite (2,7 kPa) à 40°C. On obtient ainsi 298 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β oxo-9 (pyridyl-3 carbonyl)oxy-10β taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10β méthoxy-7β oxo-9 taxène-11 yle-13α peut être préparé de la manière suivante

A une solution de 150 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β méthoxyacétoxy-10β oxo-9 taxène-11 yle-13α dans 4 cm3 d'éthanol anhydre, maintenue sous atmosphère d'argon et sous agitation, on ajoute, goutte à goutte et à une température voisine de 20°C, 0,263 cm3 d'hydrazine monohydratée. Le milieu réactionnel est maintenu sous agitation pendant 1 heure à une température voisine de 20°C puis versé dans un mélange de 100 cm3 d'acétate d'éthyle et de 50 cm3 d'eau distillée. La phase organique est séparée par décantation et on réextrait la phase aqueuse par 2 fois 50 cm3 d'acétate d'éthyle. Les phases organiques sont réunies lavées par 4 fois 50 cm3 d'eau distillée, séchées sur sulfate de magnésium, filtrées et concentrées à sec sous pression réduite (2,7 kPa) à 40°C. On obtient 180 mg d'une meringue blanche que l'on purifie par chromatographie sur gel de silice déposé sur plaques [(gel de 1 mm d'épaisseur, plaques de 20 x 20 cm, éluant : dichlorométhane-méthanol (90-10 en volumes)] par fractions de 90 mg (2 plaques). Après localisation aux rayons U.V. de la zone correspondant au produit cherché adsorbé, cette zone est grattée et la silice recueillie est lavée sur verre fritté par 10 fois 10 cm3 d'acétate d'éthyle. Les filtrats sont réunis et concentrés à sec sous pression réduite (2,7 kPa) à 40°C. On obtient ainsi 113 mg de tert-butoxycarbonyl-3 (méthoxy-4 phényl)-2 phényl-4 oxazolidine-1,3 carboxylate-5-(2R,45,5R) d'acétoxy-4α benzoyloxy-2α époxy-5β,20 dihydroxy-1β,10β méthoxy-7β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β méthoxyacétoxy-10β oxo-9 taxène-11 yle-13α peut être préparé de la manière suivante :

A une solution de 1,041 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxyacétoxy-10β méthylthiométhoxy-7β oxo-9 taxène-11 yle-13α dans 100 cm3 d'éthanol anhydre, maintenue sous atmosphère d'argon et sous agitation, on ajoute, à une température voisine de 20°C, 100 cm3 d'une suspension éthanolique de nickel activé selon Raney (obtenue à partir de 80 cm3 de la suspension aqueuse commerciale à environ 50 %, par lavage successifs jusqu'à un pH voisin de 7 par 15 fois 100 cm3 d'eau distillée et par 4 fois 150 cm3 d'éthanol). Le mélange réactionnel est maintenu sous agitation pendant 7 jours à une température voisine de 20°C puis filtré sur verre fritté. Le verre fritté est lavé par 3 fois 100 cm3 d'éthanol, les filtrats sont réunis et concentrés à sec sous pression réduite (2,7 kPa) à 40°C. On obtient 821 mg d'une meringue blanche que l'on purifie par chromatographie sur 75 g de silice (0,063-0,2 mm) contenus dans une colonne de 2,5 cm de diamètre [éluant : dichlorométhane-acétate d'éthyle (90-10 en volumes)] en recueillant des fractions de 5 cm3. Les fractions ne contenant que le produit cherché sont réunies et concentrées à sec sous pression réduite (2,7 kPa) à 40°C. On obtient ainsi 228 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β méthoxyacétoxy-10β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxyacétoxy-10β méthylthiométhoxy-7β oxo-9 taxène-11 yle-13α peut être préparé de le manière suivante :

A une solution de 5 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,7β méthoxyacétoxy-10β oxo-9 taxène-11 yle-13α dans 165 cm3 de diméthylsulfoxyde anhydre, maintenue sous atmosphère d'argon et sous agitation, on ajoute, à une température voisine de 20°C, 3,35 cm3 d'acide acétique et 11,5 cm3 d'anhydride acétique. Le mélange réactionnel est maintenu sous agitation pendant 3 jours à une température voisine de 20°C puis versé dans 500 cm3 de dichlorométhane. On additionne ensuite sous bonne agitation 100 cm3 d'une solution aqueuse saturée de carbonate de potassium jusqu'à un pH voisin de 7. Après 10 minutes d'agitation, la phase organique est séparée par décantation et on réextrait la phase aqueuse par 2 fois 250 cm3 de dichlorométhane. Les phases organiques sont réunies, lavées par 3 fois 100 cm3 d'eau distillée, séchées sur sulfate de magnésium, filtrées et concentrées à sec sous pression réduite (2,7 kPa) à 40°C. On obtient 9,5 g d'une huile jaune pâle que l'on purifie par chromatographie sur 250 g de silice (0,063-0,4 mm) contenus dans une colonne de 3 cm de diamètre [éluant : dichlorométhane-méthanol (99-1 en volumes)] en recueillant des fractions de 50 cm3. Les fractions ne contenant que le produit cherché sont réunies et concentrées à sec sous pression réduite (2,7 kPa) à 40°C. On obtient ainsi 3,01 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxyacétoxy-10β méthylthiométhoxy-7β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,7β méthoxyacétoxy-10β oxo-9 taxène-11 yle-13α peut être préparé de la manière suivante :

A une solution de 20 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 triéthylsilyloxy-7β hydroxy-1β méthoxyacétoxy-10β oxo-9 taxène-11 yle-13α dans 200 cm3 de dichlorométhane anhydre, maintenue sous atmosphère d'argon et sous agitation, on ajoute, goutte à goutte, à une température voisine de 0°C, 220 cm3 du complexe triéthylamine- acide fluorhydrique (1-3 en moles). Le mélange réactionnel est ensuite réchauffé jusqu'à une température voisine de 20°C, maintenu pendant 3 heures à cette température et versé dans 4 litres d'une solution aqueuse saturée d'hydrogénocarbonate de sodium. Le pH du milieu réactionnel étant ainsi amené aux environs de 7. Après 10 minutes d'agitation, la phase organique est séparée par décantation et on extrait la phase aqeuse par 2 fois 100 cm3 de dichlorométhane. Les phases organiques sont réunies, lavées par 100 cm3 d'eau distillée, séchées sur sulfate de magnésium, filtrées et concentrées à sec sous pression réduite (2,7 kPa) à 40°C. On obtient ainsi 17,4 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,7β méthoxyacétoxy-10β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 triéthylsilyloxy-7β hydroxy-1β méthoxyacétoxy-10β oxo-9 taxène-11 yle-13α peut être préparé dans les conditions décrites dans la demande internationale PCT WO 95/11241.

### EXEMPLE 2

En opérant comme dans l'exemple 1, mais à partir de 210 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β oxo-9 (pyridyl-2 carbonyl)oxy-10β taxène-11 yle-13α, on obtient 145 mg de tert-butoxycarbonylamino-3 hydroxy-2 pnényl-3 propionate-(2R,3S) d'acétoxy-4α benzoyloxy-2α époxy-5β,20 hydroxy-1β méthoxy-7β oxo-9 (pyridyl-2 carbonyl)oxy-10β taxène-11 yle-13α sous forme d'une meringue blanche dont les caractéristiques sont les suivantes :
- pouvoir rotatoire : [α]^{D}₂₀ = - 52 (c = 0,5 ; méthanol).
- spectre de R.M.N. ¹H (400 MHz ; CDCl₃ ; déplacements chimiques δ en ppm ; constantes de couplage J en Hz) : 1,31 (s, 3H : -CH₃ en 16 ou en 17) ; 1,37 [(s, 12H : -C(CH₃)₃ et -CH₃ en 16 ou en 17] ; 1,74 (s, 1H : -OH en 1) ; 1,78 (s, 3H : -CH₃) ; 1,82 et 2,78 (2 mts, 1H chacun : -CH₂- en 6) ; 1,97 (s, 3H : -CH₃) ; 2,35 (d, J = 9, 2H : -CH₂- en 14) ; 2,40 (s, 3H : -COCH₃) ; 3,40 (d, J = 4,5, 1H : -OH en 2') ; 3,43 (s, 3H : -OCH₃) ; 3,92 (d, J = 7,5, 1H : -H en 3) ; 3,98 (dd, J = 11 et 7, 1H : -H en 7); 4,20 et 4,32 (2 d, J = 8,5, 1H chacun : -CH₂- en 20) ; 4,64 (mt, 1H : -H en 2'); 5,00 (d large, J = 10, 1H : -H en 5) ; 5,28 (d large, J = 10, 1H : -H en 3') ; 5,43 (d, J = 10, 1H : -CONH-) ; 5,73 (d, J = 7,5, 1H : -H en 2) ; 6,22 (t large, J = 9, 1H : -H en 13) ; 6,67 (s, 1H : -H en 10) ; de 7,25 à 7,45 (mt, 5H : -C₆H₅ en 3') ; 7,51 [(mt, 3H : -OCOC₆H₅(-H en 3 et H en 5) et -OCOC₅H₄N(-H en 5)] ; 7,61 [(t, J = 7,5, 1H : -OCOC₆H₅(-H en 4)] ; 7,88 [(t dédoublé, J = 8 et 1, 1H : -OCOC₅H₄N(-H en 4)]; 8,12 [(d, J = 7,5, 2H : -OCOC₆H₅(-H en 2 et -H en 6)] ; 8,20 (d large, J = 8, 1H : -OCOC₅H₄N(-H en 3)]; 8,82 (dd large J = 5 et 1, 1H : -OCOC₅H₄N(-H en 6)].

En opérant comme dans l'exemple 1, mais à partir de 300 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10β méthoxy-7β oxo-9 taxène-11 yle-13α, on obtient 230 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β oxo-9 (pyridyl-2 carbonyl)oxy-10β taxène-11 yle-13α sous forme d'une meringue blanche.

### EXEMPLE 3

En opérant comme dans l'exemple 1, mais à partir de 300 mg de tert-butoxycarbonyl-3 (méthoxy-4 phényl)-2 phényl4 oxazolidine-1,3 carboxylate-5-(2R,4S,5R) d'acétoxy-4α benzoyloxy-2α cydopentylcarbonyloxy-10β époxy-5β,20 hydroxy-1β méthoxy-7β oxo-9 taxène-11 yle-13α, on obtient 96 mg de tert-butoxycarbonylamino-3 hydroxy-2 phényl-3 propionate-(2R,3S) d'acétoxy-4α benzoyloxy-2α cyclopentylcarbonyloxy-10β époxy-5β,20 hydroxy-1β méthoxy-7β oxo-9 taxène-11 yle-13a sous forme d'une meringue blanche dont les caractéristiques sont les suivantes :
- pouvoir rotatoire : [α]^{D}₂₀ = - 66 (c = 0,5 ; méthanol).
- spectre de R.M.N. ¹H (400 MHz ; CDCl₃ ; déplacements chimiques δ en ppm ; constantes de couplage J en Hz) : 1,25 (s, 6H : -CH₃ en 16 et en 17) ; 1,39 [s, 9H : -C(CH₃)₃] ; de 1,55 à 1,80 et de 1,90 à 2,10 (2 mts, 4H chacun : -CH₂- du cyclopentyl) ; 1,71 (s, 1H : -OH en 1) ; 1,75 (s, 3H : -CH₃) ; 1,82 et 2,75 (2 mts, 1H chacun : -CH2- en 6) ; 1,93 (s, 3H : -CH3) ; 2,33 (d, J = 9 Hz, 2H : -CH₂- en 14) ; 2,39 (s, 3H : -COCH₃) ; 2,95 (mt, 1H : =CH- du cyclopentyl) ; 3,38 (s, 3H : -OCH₃); 3,40 (d, J = 5, 1H : -OH en 2') ; 3,88 (d, J = 7,5, 1H : -H en 3) ; 3,91 (dd, J = 11 et 7,5, 1H : -H en 7) ; 4,19 et 4,32 (2 d, J = 8,5, ¹H chacun : -CH₂- en 20) ; 4,65 (mt, 1H : -H en 2°) ; 4,98 (d large, J = 10, 1H : -H en 5) ; 5,28 (d large, J = 10, 1H : -H en 3') ; 5,41 (d, J = 10, 1H : -CONH-) ; 5,68 (d, J = 7,5, 1H : -H en 2) ; 6,21 (t large, J = 9, 1H : -H en 13) ; 6,45 (s, 1H : -H en 10) ; de 7,25 à 7,45 (mt, 5H : -C₆H₅ en 3'); 7,51 [t, J = 7,5, 2H : -OCOC₆H₅ (-H en 3 et H en 5)] ; 7,63 [t, J = 7,5, 1H : -OCOC₆H₅ (-H en 4)] ; 8,12 [d, J = 7,5, 2H : -OCOC₆H₅ (-H en 2 et -H en 6)].

En opérant comme dans l'exemple 1, mais à partir de 300 mg de tert-butoxycarbonyl-3 (méthoxy-4 phényl)-2 phénol-4 oxazolidine-1,3 carboxylate-5-(2R,4S,5R) d'acétoxy-4α benzoyloxy-2α époxy-5β,20 dihydroxy-1β,10β méthoxy-7β oxo-9 taxène-11 yle-13α, on obtient 410 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α cyclopentylcarbonyloxy-10β époxy-5β,20 hydroxy-1β méthoxy-7β oxo-9 taxène-11 yle-13α sous forme d'une meringue blanche.

### EXEMPLE 4

En opérant comme dans l'exemple 1, mais à partir de 300 mg de tert-butoxycarbonyl-3 (méthoxy-4 phényl)-2 phénol-4 oxazolidine-1,3 carboxylate-5-(2R,4S,5R) d'acétoxy-4α benzoyloxy-2α cyclopropylcarbonyloxy-10β époxy-5β,20 hydroxy-1β méthoxy-7β oxo-9 taxène-11 yle-13α, on obtient 130 mg de tert-butoxycarbonylamino-3 hydroxy-2 phényl-3 propionate-(2R,3S) d'acétoxy-4α benzoyloxy-2α cyclopropylcarbonyloxy-10β époxy-5β,20 hydroxy-1β méthoxy-7β oxo-9 taxène-11 yle-13α sous forme d'une meringue blanche dont les caractéristiques sont les suivantes :
- pouvoir rotatoire : [α]^{D}₂₀ = -71 (c = 0,5 ; méthanol).
- spectre de R.M.N. ¹H (400 MHz ; CDCl₃, déplacements chimiques δ en ppm ; constantes de couplage J en Hz) : 1,00 et 1,19 (2 mts, 2H chacun : -CH₂- du cyclopropyl) ; 1,25 (s, 3H : -CH₃ en 16 ou en 17) ; 1,27 (s, 3H : -CH₃ en 16 ou en 17 ; 1,39 [s, 9H : -C(CH₃)₃] ; 1,71 (s, 1H : -OH en 1) ; 1,75 (s, 3H : -CH₃) ; de 1,70 à 1,90 (mt, 1H : =CH- du cyclopropyl) ; 1,82 et 2,75 (2 mts, 1H chacun : -CH₂- en 6) ; 1,93 (s, 3H : -CH₃) ; 2,33 (d, J = 9, 2H : -CH₂- en 14) ; 2,40 (s, 3H : -COCH₃) ; 3,35 (s, 3H : -OCH₃) ; 3,40 (d, J = 5, 1H : -OH en 2'); 3,88 (d, J = 7,5, 1H : -H en 3) ; 3,89 (dd, J = 11 et 7,5, 1H : -H en 7) ; 4,19 et 4,32 (2 d, J = 8,5, 1H chacun : -CH₂- en 20) ; 4,65 (mt, 1H : -H en 2') ; 5,00 (d large, J = 10, 1H : -H en 5) ; 5,28 (d large, J = 10, 1H : -H en 3') ; 5,42 (d, J = 10, 1H : -CONH- ) ; 5,68 (d, J = 7,5, 1H : -H en 2) ; 6,21 (t large, J = 9, 1H : -H en 13) ; 6,48 (s, 1H : -H en 10) ; de 7,25 à 7,45 (mt, 5H : -C₆H₅ en 3') ; 7,52 [t, J = 7,5, 2H : -OCOC₆H₅ (-H en 3 et H en 5)] ; 7,64 [t, J = 7,5, 1H : -OCOC₆H₅ (-H en 4)] ; 8,12 [d, J = 7,5, 2H : -OCOC₆H₅ (-H en 2 et -H en 6)].

En opérant comme dans l'exemple 1, mais à partir de 300 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10β méthoxy-7β oxo-9 taxène-11 yle-13a, on obtient 435 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α cyclopropylcarbonyloxy-10β époxy-5β,20 hydroxy-1β méthoxy-7β oxo-9 taxène-11 yle-13α sous forme d'une meringue blanche.

### EXEMPLE 5

En opérant comme dans l'exemple 1, mais à partir de 430 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β méthoxyacétoxy-10β oxo-9 taxène-11 yle-13α, on obtient 164 mg de de tert-butoxycarbonylamino-3 hydroxy-2 phényl-3 propionate-(2R,3S) d'acétoxy-4α benzoyloxy-2α époxy-5β,20 hydroxy-1β méthoxy-7β méthoxyacétoxy-10β oxo-9 taxène-11 yle-13α sous forme d'une meringue blanche dont les caractéristiques sont les suivantes :
- pouvoir rotatoire : [α]^{D}₂₀ = - 48 (c = 0,5 ; méthanol)
- spectre de R.M.N. ¹H (300 MHz ; CDCl₃ ; δ en ppm ; constantes de couplage J en Hz) : 1,17 (s, 3H : -CH₃) ; 1,22 (s, 3H : -CH₃) ; 1,35 (s, 9H : -C(CH₃)₃ ; 1,75 (s, 3H : -CH₃) ; 1,80 et 2,75 (2 mts, 1H chacun : -CH₂- 6); 1,90 (s, 3H : -CH₃) ; 2,30 (d, J = 9, 2H : -CH₂- 14) ; 2,37 (s, 3H : -COCH₃) ; 3,35 et 3,55 (2 s, 3H chacun : -OCH₃) ; 3,40 (d, J = 5, 1H : -OH 2') ; 3,85 (d, J = 7, 1H : -H 3) ; 3,88 (dd, J = 11 et 7, 1H : -H 7) ; 4,17 et 4,32 (2 d, J = 8,5, 1H chacun : -CH₂- 20); 4,19 et 4,27 (2 d, J = 15, 1H chacun : -OCOCH₂OCH₃) ; 4,65 (mt, 1H : -H 2'); 4,97 (d large, J = 10, 1H : -H 5) ; 5,25 (d large, J = 10, 1H : -H 3') ; 5,42 (d, J = 10, 1H : -CONH -) ; 5,66 (d, J = 7, 1H : -H 2) ; 6,18 (t large, J = 9, 1H : -H 13) ; 6,52 (s, 1H : -H 10) ; de 7,30 à 7,50 (mt, 5H : -C₆H₅ 3') ; 7,51 [(t, J = 7,5, 2H : -OCOC₆H₅(-H 3 et H 5)] ; 7,63 [(t, J = 7,5, 1H : -OCOC₆H₅(-H 4)] ; 8,12 (d, J = 7,5, 2H : -OCOC₆H₅(-H 2 et H 6)].

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants. Cependant, les compositions peuvent aussi se présenter sous forme de comprimés, de pilules, de poudres ou de granulés administrables par voie orale.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérilisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques , des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées telles que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogènes comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. 11 est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Nouveaux taxoïdes de formule générale : 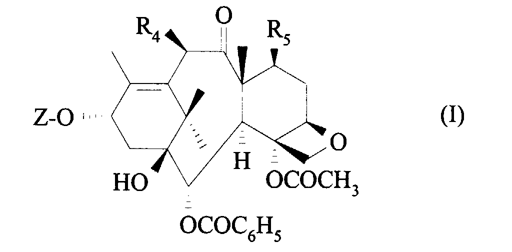 dans laquelle
Z représente un atome d'hydrogène ou un radical de formule générale : 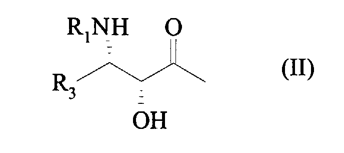 dans laquelle :
R₁ représente
- un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle
- ou un radical R₂-O-CO- dans lequel R₂ représente :
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,
R₃ représente :
- un radical alcoyle droit ou ramifié contenant 1 à 8 atomes de carbone.
- alcényle droit ou ramifié contenant 2 à 8 atomes de carbone,
- alcynyle droit ou ramifié contenant 2 à 8 atomes de carbone,
- cycloalcoyle contenant 3 à 6 atomes de carbone,
- phényle ou α- ou β-naphtyle
éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro et trifluorométhyle,
- ou un hétérocycle aromatique ayant 5 chaînons et contenant un ou plusieurs hétéroatomes, identiques ou différents, choisis parmi les atomes d'azote, d'oxygène ou de soufre et
éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano. carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
R₄ représente :
- un radical alcanoyloxy dont la partie alcanoyle contient 2 à 6 atomes de carbone en chaîne droite ou ramifiée, à l'exception du radical acétoxy,
- alcénoyloxy dont la partie alcénoyle contient 3 à 6 atomes de carbone en chaîne droite ou ramifiée,
- alcynoyloxy dont la partie alcynoyle contient 3 à 6 atomes de carbone en chaîne droite ou ramifiée,
ces radicaux étant éventuellement substitués par un ou plusieurs atomes d'halogène ou par un radical alcoxy contenant 1 à 4 atomes de carbone, alcoylthio contenant 1 à 4 atomes de carbone, ou par un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical cycloalcanoyloxy dont la partie cycloalcanoyle contient 4 à 8 atomes de carbone ou un radical cycloalcénoyloxy dont la partie cycloalcénoyle contient 4 à 8 atomes de carbone, ou bien R₄ représente un radical benzoyloxy ou hétérocyclylcarbonyloxy dans lequel la partie hétérocyclique représente un hétérocycle aromatique à 5 ou 6 chaînons contenant un ou plusieurs hétéroatomes choisis parmi les atomes d'oxygène, de soufre ou d'azote,
R₅ représente :
- un radical alcoxy contenant 1 à 6 atomes ce carbone en chaîne droite ou ramifiée éventuellement substitué par un radical alcoxy contenant 1 à 4 atomes de carbone,
- alcényloxy contenant 3 à 6 atomes de carbone,
- alcynyloxy contenant 3 à 6 atomes de carbone,
- cycloalcoyloxy contenant 3 à 6 atomes de carbone,
- cycloalcényloxy contenant 3 à 6 atomes de carbone,
ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone.

2. Nouveaux taxoïdes selon la revendication 1 pour lesquels
Z représente un atome d'hydrogène ou un radical de formule générale (Il) dans laquelle R₁ représente un radical benzoyle ou un radical R₂-O-CO- dans lequel R₂ représente un radical tert-butyle
et R₃ représente :
- un radical alcoyle contenant 1 à 6 atomes de carbone,
- alcényle contenant 2 à 6 atomes de carbone,
- cycloalcoyle contenant 3 à 6 atomes de carbone,
- phényle
éventuellement substitué par un ou plusieurs atomes ou radicaux, identiques ou différents choisis parmi les atomes d'halogène et les radicaux alcoyles , alcoxy, dialcoylamino, acylamino, alcoxycarbonylamino ou trifluorométhyle
- ou un radical furyle-2 ou -3,
- thiényle-2 ou -3 ou
- thiazolyle-2, -4 ou -5 et
R₄ représente un radical alcoyloxyacétoxy dont la partie alcoyle contient 1 à 4 atomes de carbone, cycloalcanoyloxy dont la partie cycloalcanoyle contient 4 à 8 atomes de carbone, pyridylcarbonyloxy et R₅ représente un radical alcoyloxy droit ou ramifié contenant 1 à 6 atomes de carbone.

3. Nouveaux taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ représente un radical méthoxyacétoxy, cyclopropylcarbonyloxy, cyclopentylcarbonyloxy, pyridyl-2 carbonyloxy ou pyridyl-3 carbonyloxy et R₅ représente un radical méthoxy.

4. Procédé de préparation des taxoïdes selon l'une des revendications 1, 2 ou 3 pour lequel Z représente un radical de formule générale (II) caractérisé en ce que l'on estérifie un produit de formule générale : 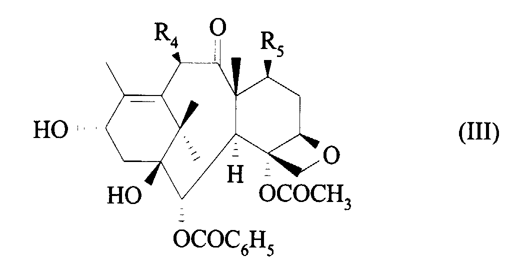 dans laquelle R₄ et R₅ sont définis comme dans l'une des revendications 1, 2 ou 3, au moyen d'un acide de formule générale : 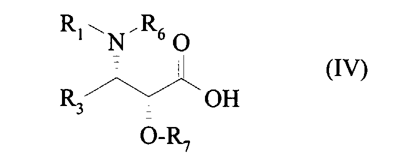 dans laquelle R₁ et R₃ sont définis comme précédemment, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 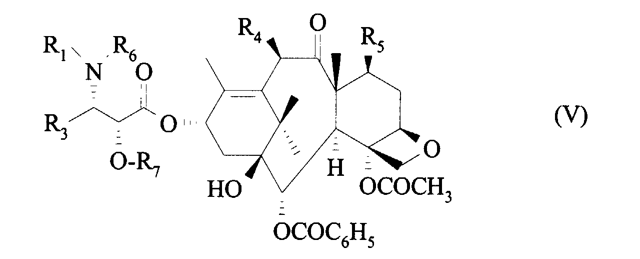 dans laquelle R₁, R₃, R₄, R₅, R₆ et R₇ sont définis comme précédemment, dont on remplace les groupements protecteurs représentés par R₇ et/ou R₆ et R₇ par des atomes d'hydrogène.

5. Procédé selon la revendication 4 caractérisé en ce que l'estérification est effectuée au moyen d'un acide de formule générale (IV) en présence d'un agent de condensation et d'un agent d'activation dans un solvant organique à une température comprise entre -10 et 90°C.

6. Procédé selon la revendication 4 caractérisé en ce que l'estérification est effectuée au moyen d'un acide de formule générale (IV) sous forme d'anhydride symétrique en opérant en présence d'un agent d'activation dans un solvant organique à une température comprise entre 0 et 90°C.

7. Procédé selon la revendication 4 caractérisé en ce que l'estérification est effectuée en utilisant l'acide de formule générale (IV) sous forme d'halogénure ou sous forme d'anhydride mixte avec un acide aliphatique ou aromatique, éventuellement préparé in situ, en présence d'une base en opérant dans un solvant organique à une température comprise entre 0 et 80°C.

8. Procédé selon la revendication 4 caractérisé en ce que l'on remplace les groupements protecteurs R₇ et/ou R₆ et R₇ par des atomes d'hydrogène en opérant, selon leur nature de la manière suivante :
1) lorsque R₆ représente un atome d'hydrogène et R₇ représente un groupement protecteur de la fonction hydroxy, on remplace les groupements protecteurs par des atomes d'hydrogène au moyen d'un acide minéral ou organique utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₆ et R₇ forment ensemble un hétérocycle saturé à 5 ou 6 chaînons de formule générale : 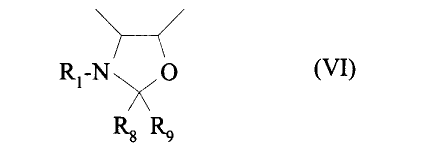 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, on remplace le groupement protecteur formé par R₆ et R₇ par des atomes d'hydrogène en opérant, selon les significations de R₁, R₈ et R₉, de la manière suivante :
a) lorsque R₁ représente un radical tert-butoxycarbonyle, R₈ et R₉, identiques ou différents, représentent un radical alcoyle ou un radical aralcoyle ou aryle, ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, on traite l'ester de formule générale (V) par un acide minéral ou organique éventuellement dans un solvant organique tel qu'un alcool pour obtenir le produit de formule générale : 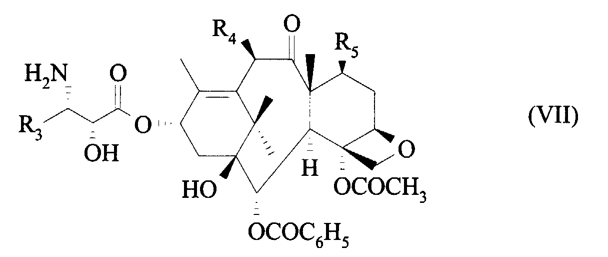 dans laquelle R₃, R₄ et R₅ sont définis comme précédemment, que l'on acyle au moyen de chlorure de benzoyle dans lequel le noyau phényle est éventuellement substitué, de chlorure de thénoyle, de chlorure de furoyle ou d'un produit de formule générale :
R₂-O-CO-X (VIII)
dans laquelle R₂ est défini comme précédemment et X représente un atome d'halogène ou un reste -O-R₂ ou -O-CO-O-R₂, pour obtenir un produit de formule générale (I) dans laquelle Z représente un radical de formule générale (II),
b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, on remplace le groupement protecteur formé par R₆ et R₇ par des atomes d'hydrogène en présence d'un acide minéral ou organique utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C.

9. Procédé de préparation d'un nouveau taxoïde selon l'une des revendications 1, 2 ou 3 pour lequel Z représente un atome d'hydrogène, R₄ est défini comme dans l'une des revendications 1, 2 ou 3 et R₅ est défini comme dans l'une des revendications 1, 2 ou 3 caractérisé en ce que l'on traite la 10-désacétylbaccatine III de formule : 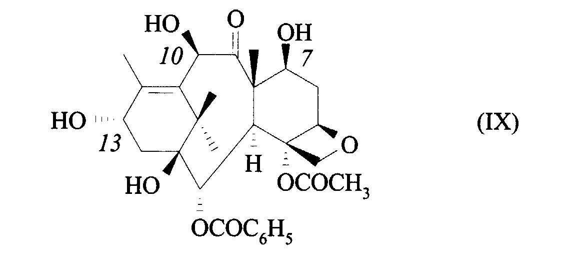 par un halogénure de silyle de formule générale :
(R)₃-Si-Hal (X)
dans laquelle les symboles R, identiques ou différents, représentent un radical alcoyle contenant 1 à 4 atomes de carbone éventuellement substitué par un radical phényle, un radical cycloalcoyle contenant 3 à 6 atomes de carbone ou un radical phényle pour obtenir un produit de formule générale : 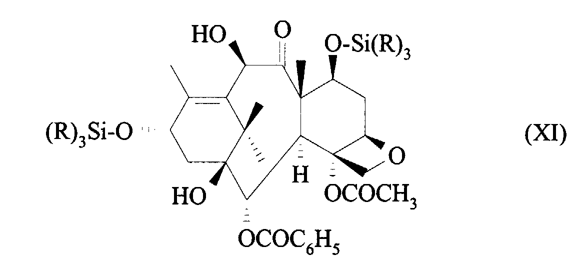 dans laquelle R est défini comme précédemment, que l'on traite par un produit de formule générale :
R'₄-X₁ (XII)
dans laquelle R'₄ est tel que R'₄-O- est identique à R₄ défini comme dans l'une des revendications 1, 2 ou 3 et X₁ représente un atome d'halogène pour obtenir un produit de formule générale : 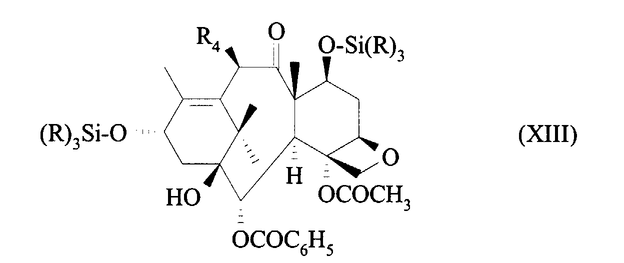 dans laquelle R et R₄ sont définis comme précédemment, dont on remplace les groupements protecteurs silylés par des atomes d'hydrogène pour obtenir un produit de formule générale : 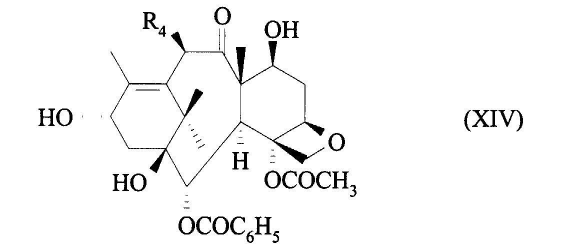 dans laquelle R₄ est défini comme précédemment, qui est éthérifié sélectivement en position 7 par action d'un produit de formule générale :
R'₅-X₂ (XV)
dans laquelle R'₅ est tel que R'₅-O- est identique à R₅ défini comme dans l'une des revendications 1, 2 ou 3 et X₂ représente un reste d'ester réactif ou un atome d'halogène pour donner le produit de formule générale (I) dans laquelle Z représente un atome d'hydrogène.

10. Procédé de préparation d'un produit selon l'une des revendications 1, 2 ou 3 pour lequel Z représente un radical de formule générale (II), R₄ est défini comme dans l'une des revendications 1, 2 ou 3 et R₅ est défini comme dans l'une des revendications 1, 2 ou 3 caractérisé en ce que l'on traite un produit de formule générale : 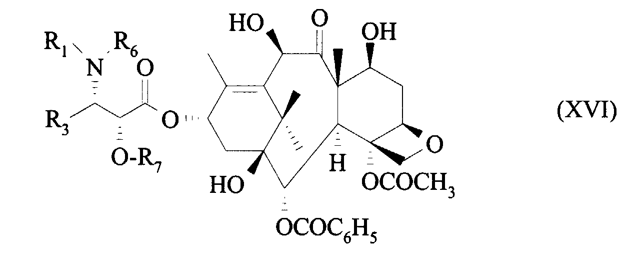 dans laquelle R₁, R₃, R₆ et R₇ sont définis comme dans l'une des revendications 1, 2, 3 ou 4 au moyen d'un produit de formule générale :
(R)₃Si-Hal (X)
dans laquelle les symboles R, identiques ou différents, représentent un radical alcoyle contenant 1 à 4 atomes de carbone éventuellement substitué par un radical phényle, ou un radical cycloalcoyle contenant 3 à 6 atomes de carbone, ou un radical phényle pour obtenir un produit de formule générale : 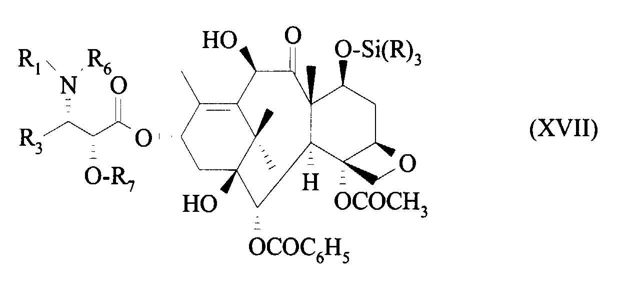 dans laquelle R, R₁, R₃, R₆ et R₇ sont définis comme précédemment, qui est fonctionnalisé en position 10 au moyen d'un produit de formule générale :
R'₄-X₁ (XII)
dans laquelle R₄ est tel que R'₄-O- est identique à R₄ défini comme dans l'une des revendications 1, 2 ou 3 et X₁ représente un atome d'halogène ou un reste d'ester réactif pour donner un produit de formule générale : 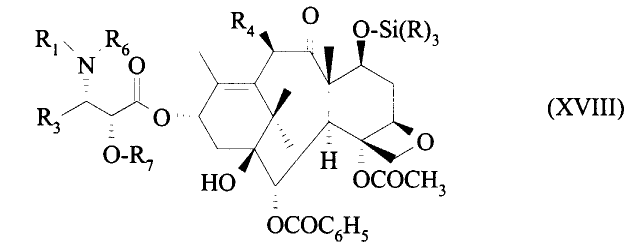 dans laquelle R, R₁, R₃, R₄, R₆ et R₇ sont définis comme précédemment dont le groupement protecteur silylé est remplacé par un atome d'hydrogène pour donner un produit de formule générale : 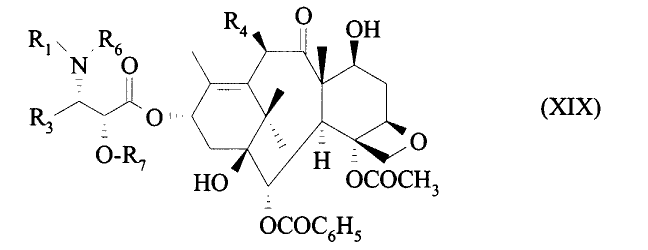 qui, par action d'un produit de formule générale (XV) conduit au produit de formule générale (V) dont les groupements protecteurs sont remplacés par des atomes d'hydrogène pour donner un produit de formule générale (I) dans laquelle Z représente un radical de formule générale (II).

11. Procédé de préparation d'un produit selon l'une des revendications 1, 2 ou 3, caractérisé en ce que l'on fait réagir du nickel Raney activé en présence d'un alcool aliphatique contenant 1 à 3 atomes de carbone ou d'un éther sur un produit de formule générale : 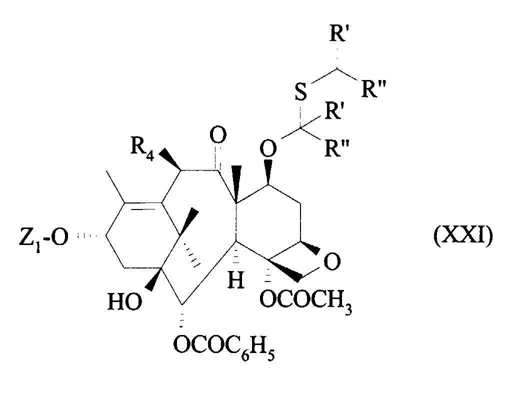 dans laquelle R₄ est défini comme dans l'une des revendications 1, 2 ou3, R' et R", identiques ou différents, représentent un atome d'hydrogène ou un radical alcoyle contenant 1 à 6 atomes de carbone, alcényle contenant 2 à 6 atomes de carbone, alcynyle contenant 3 à 6 atomes de carbone, cycloalcoyle contenant 2 à 6 atomes de carbone ou cycloalcényle contenant 3 à 6 atomes de carbone éventuellement substitué, ou bien R' et R" forment ensemble avec l'atome de carbone auquel ils sont liés un radical cycloalcoyle contenant 3 à 6 atomes de carbone ou un radical cycloalcényle contenant 4 à 6 atomes de carbone, et Z₁ représente un atome d'hydrogène ou un radical de formule générale : 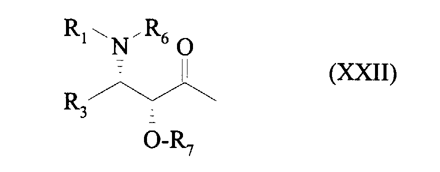 dans laquelle R₁ et R₃ sont définis comme dans l'une des revendications 1 à 3 et R₆ et R₇ sont définis comme dans la revendication 4, pour obtenir un produit de formule générale : 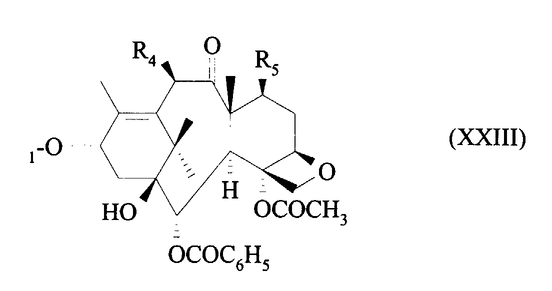 suivi, lorsque Z₁ représente un radical de formule générale (XXII), du remplacement des groupements protecteurs représentés par R₆ et/ou R₆ et R₇ par des atomes d'hydrogène dans les conditions de la revendication 8.

12. Procédé de préparation selon la revendication 11 caractérisé en ce que l'on opére à une température comprise entre -10 et 60°C.

13. Le tert-butoxycarbonylamino-3 hydroxy-2 phényl-3 propionate-(2R,3S) d'acétoxy-4α benzoyloxy-2α époxy-5β,20 hydroxy-1β méthoxy-7β méthoxyacétoxy-10β oxo-9 taxène-11 yle-13α.

14. Le tert-butoxycarbonylamino-3 hydroxy-2 phényl-3 propionate-(2R,3S) d'acétoxy-4α benzoyloxy-2α hydroxy-1β époxy-5β,20 méthoxy-7β cyclopropylcarbonyloxy-10β oxo-9 taxène-11 yle-13α.

15. Le tert-butoxycarbonylamino-3 hydroxy-2 phényl-3 propionate-(2R,3S) d'acétoxy-4α benzoyloxy-2α hydroxy-1β époxy-5β,20 méthoxy-7β cyclopentylcarbonyloxy-10β oxo-9 taxène-11 yle-13α.

16. Le tert-butoxycarbonylamino-3 hydroxy-2 phényl-3 propionate-(2R,3S) d'acétoxy-4α benzoyloxy-2α hydroxy-1β époxy-5β,20 méthoxy-7β (pyridyl-2)carbonyloxy-10β oxo-9 taxène-11 yle-13α.

17. Le tert-butoxycarbonylamino-3 hydroxy-2 phényl-3 propionate-(2R,3S) d'acétoxy-4α benzoyloxy-2α hydroxy-1β époxy-5β,20 méthoxy-7β (pyridyl-3)carbonyloxy-10β oxo-9 taxène-11 yle-13α.

18. Composition pharmaceutique caractérisée en ce qu'elle contient au moins un produit selon l'une des revendications 1, 2 ou 3 pour lequel Z représente un radical de formule générale (II) en association avec un ou plusieurs diluants ou adjuvants pharmaceutiquement acceptables et éventuellement un ou plusieurs composés compatibles et pharmacologiquement actifs.

19. Composition pharmaceutique caractérisée en ce qu'elle contient au moins le produit selon la revendication 13 en association avec un ou plusieurs diluants ou adjuvants pharmaceutiquement acceptables et éventuellement un ou plusieurs composés compatibles et pharmacologiquement actifs.

20. Composition pharmaceutique caractérisée en ce qu'elle contient au moins le produit selon la revendication 14 en association avec un ou plusieurs diluants ou adjuvants pharmaceutiquement acceptables et éventuellement un ou plusieurs composés compatibles et pharmacologiquement actifs.

21. Composition pharmaceutique caractérisée en ce qu'elle contient au moins le produit selon la revendication 15 en association avec un ou plusieurs diluants ou adjuvants pharmaceutiquement acceptables et éventuellement un ou plusieurs composés compatibles et pharmacologiquement actifs.

22. Composition pharmaceutique caractérisée en ce qu'elle contient au moins le produit selon la revendication 16 en association avec un ou plusieurs diluants ou adjuvants pharmaceutiquement acceptables et éventuellement un ou plusieurs composés compatibles et pharmacologiquement actifs

23. Composition pharmaceutique caractérisée en ce qu'elle contient au moins le produit selon la revendication 17 en association avec un ou plusieurs diluants ou adjuvants pharmaceutiquement acceptables et éventuellement un ou plusieurs composés compatibles et pharmacologiquement actifs.

## Patentansprüche

1. Neue Taxoide der allgemeinen Formel (I) 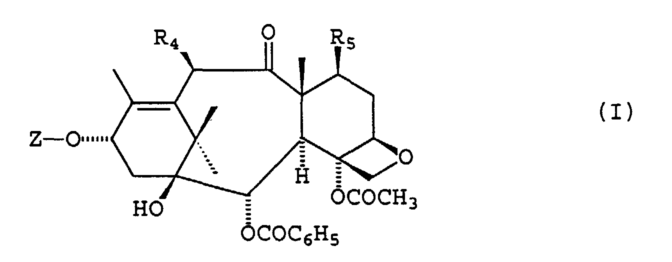 in der
Z ein Wasserstoffatom oder einen Rest der allgemeinen Formel (II) 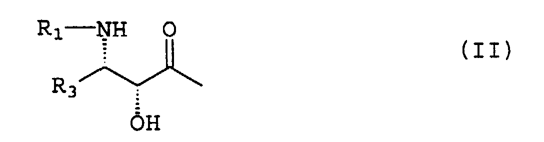 bedeutet, worin
R₁ darstellt
- einen Rest Benzoyl, gegebenenfalls substituiert durch ein oder mehrere, gleiche oder verschiedene Atome oder Reste, ausgewählt unter den Halogenatomen und den Resten Alkyl mit 1 bis 4 Kohlenstoffatomen, Alkoxy mit 1 bis 4 Kohlenstoffatomen oder Trifluormethyl, Thenoyl oder Furoyl,
- oder einen Rest R₂-O-CO-, worin R₂ bedeutet:
- einen Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen, Bicycloalkyl mit 7 bis 10 Kohlenstoffatomen, wobei diese Reste gegebenenfalls durch einen oder mehrere Substituenten substituiert sind, ausgewählt unter den Halogenatomen und den Resten Hydroxy, Alkoxy mit 1 bis 4 Kohlenstoffatomen, Dialkylamino, bei dem jeder Teil Alkyl 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Teil Alkyl 1 bis 4 Kohlenstoffatome enthält), Cycloalkyl mit 3 bis 6 Kohlenstoffatomen, Cycloalkenyl mit 4 bis 6 Kohlenstoffatomen, Phenyl, (gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen), Cyano, Carboxy oder Alkoxycarbonyl, dessen Teil Alkyl 1 bis 4 Kohlenstoffatome enthält,
- einen Rest Phenyl oder α- oder β-Naphthyl, gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen oder einem aromatischen heterocyclischen Rest mit 5 Ringgliedern, vorzugsweise ausgewählt unter den Resten Furyl und Thienyl,
- oder einen gesättigten heterocyclischen Rest mit 4 bis 6 Kohlenstoffatomen, gegebenenfalls substituiert durch einen oder mehrere Reste Alkyl mit 1 bis 4 Kohlenstoffatomen,
R₃ darstellt:
- einen geraden oder verzweigten Rest Alkyl mit 1 bis 8 Kohlenstoffatomen,
- einen geraden oder verzweigten Rest Alkenyl mit 2 bis 8 Kohlenstoffatomen,
- einen geraden oder verzweigten Rest Alkinyl mit 2 bis 8 Kohlenstoffatomen,
- Cycloalkyl mit 3 bis 6 Kohlenstoffatomen,
- Phenyl oder α- oder β-Naphthyl,
gegebenenfalls substituiert durch ein oder mehrere Atome oder Reste, ausgewählt unter den Halogenatomen und den Resten Alkyl, Alkenyl, Alkinyl, Aryl, Aralkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Alkylcarbamoyl, Dialkylcarbamoyl, Cyano, Nitro und Trifluormethyl,
- oder einen aromatischen Heterocyclus mit 5 Ringgliedern und einen oder mehreren, gleichen oder verschiedenen Heteroatomen, ausgewählt unter den Atomen von Stickstoff, Sauerstoff oder Schwefel, und gegebenenfalls substituiert durch ein oder mehrere, gleiche oder verschiedene Substituenten, ausgewählt unter den Halogenatomen und den Resten Alkyl, Aryl, Amino, Alkylamino, Dialkylamino, Alkoxycarbonylamino, Acyl, Arylcarbonyl, Cyano, Carboxy, Carbamoyl, Alkylcarbamoyl, Dialkylcarbamoyl oder Alkoxycarbonyl, mit der Maßgabe, daß bei den Substituenten der Reste Phenyl, α- oder β-Naphthyl und der aromatischen Heterocyclen die Reste Alkyl und die Teile Alkyl der anderen Reste 1 bis 4 Kohlenstoffatome und die Reste Alkenyl und Alkinyl 2 bis 8 Kohlenstoffatome enthalten und daß die Reste Aryl Reste Phenyl oder α- oder β-Naphthyl sind,
R₄ darstellt:
- einen Rest Alkanoyloxy, dessen Teil Alkanoyl 2 bis 6 Kohlenstoffatome in gerader oder verzweigter Kette enthält, mit Ausnahme des Restes Acetoxy,
- Alkenoyloxy, dessen Teil Alkenoyl 3 bis 6 Kohlenstoffatome in gerader oder verzweigter Kette enthält,
- Alkinoyloxy, dessen Teil Alkinoyl 3 bis 6 Kohlenstoffatome in gerader oder verzweigter Kette enthält,
wobei diese Reste gegebenenfalls substituiert sind durch ein oder mehrere Halogenatome oder durch einen Rest Alkoxy mit 1 bis 4 Kohlenstoffatomen, Alkylthio mit 1 bis 4 Kohlenstoffatomen, oder durch einen Rest Carboxy, Alkyloxycarbonyl, dessen Teil Alkyl 1 bis 4 Kohlenstoffatome enthält, Cyano, Carbamoyl, N-Alkylcarbamoyl oder N,N-Dialkylcarbamoyl, bei dem jeder Teil Alkyl 1 bis 4 Kohlenstoffatome enthält oder zusammen mit dem Stickstoffatom, an das sie gebunden sind, einen gesättigten heterocyclischen Rest mit 5 oder 6 Ringgliedern und gegebenenfalls einem zweiten Heteroatom, ausgewählt unter den Atomen von Sauerstoff, Schwefel oder Stickstoff, bilden, gegebenenfalls substituiert durch einen Rest Alkyl mit 1 bis 4 Kohlenstoffatomen oder einen Rest Phenyl oder einen Rest Phenylalkyl, dessen Teil Alkyl 1 bis 4 Kohlenstoffatome enthält, oder auch R₄ einen Rest Cycloalkanoyloxy, dessen Teil Cycloalkanoyl 4 bis 8 Kohlenstoffatome enthält oder einen Rest Cycloalkenoyloxy, dessen Teil Cycloalkenoyl 4 bis 8 Kohlenstoffatome enthält, darstellt, oder auch R₄ ein Rest Benzoyloxy oder Heterocyclylcarbonyloxy ist, bei dem der heterocyclische Teil einen aromatischen Heterocyclus mit 5 oder 6 Ringgliedern und einem oder mehreren Heteroatomen, ausgewählt unter den Atomen von Sauerstoff, Stickstoff oder Schwefel, bedeutet,
R₅ darstellt
- einen Rest Alkoxy mit 1 bis 6 Kohlenstoffatomen in gerader oder verzweigter Kette, gegebenenfalls substituiert durch einen Rest Alkoxy mit 1 bis 4 Kohlenstoffatomen,
- Alkenyloxy mit 3 bis 6 Kohlenstoffatomen,
- Alkinyloxy mit 3 bis 6 Kohlenstoffatomen,
- Cycloalkyloxy mit 3 bis 6 Kohlenstoffatomen,
- Cycloalkenyloxy mit 3 bis 6 Kohlenstoffatomen,
wobei diese Reste gegebenenfalls substituiert sind durch ein oder mehrere Halogenatome oder durch einen Rest Alkoxy mit 1 bis 4 Kohlenstoffatomen, Alkylthio mit 1 bis 4 Kohlenstoffatomen, oder durch einen Rest Carboxy, Alkyloxycarbonyl, dessen Teil Alkyl 1 bis 4 Kohlenstoffatome enthält, Cyano, Carbamoyl, N-Alkylcarbamoyl oder N,N-Dialkylcarbamoyl, bei dem jeder Teil Alkyl 1 bis 4 Kohlenstoffatome enthält oder zusammen mit dem Stickstoffatom, an das sie gebunden sind, einen gesättigten heterocyclischen Rest mit 5 oder 6 Ringgliedern und gegebenenfalls einem zweiten Heteroatom, ausgewählt unter den Atomen von Sauerstoff, Schwefel oder Stickstoff, bilden, gegebenenfalls substituiert durch einen Rest Alkyl mit 1 bis 4 Kohlenstoffatomen oder einen Rest Phenyl oder einen Rest Phenylalkyl, dessen Teil Alkyl 1 bis 4 Kohlenstoffatome enthält.

2. Neue Taxoide nach Anspruch 1, worin
Z ein Wasserstoffatom oder einen Rest der allgemeinen Formel (II) bedeutet, in der R₁ einen Rest Benzoyl oder einen Rest R₂-O-CO- darstellt, worin R₂ ein Rest tert.-Butyl ist, und
R₃ darstellt
- einen Rest Alkyl mit 1 bis 6 Kohlenstoffatomen,
- Alkenyl mit 2 bis 6 Kohlenstoffatomen,
- Cycloalkyl mit 3 bis 6 Kohlenstoffatomen,
- Phenyl,
gegebenenfalls substituiert durch ein oder mehrere, gleiche oder verschiedene Atome oder Reste, ausgewählt unter den Halogenatomen und den Resten Alkyl, Alkoxy, Dialkylamino, Acylamino, Alkoxycarbonylamino oder Trifluormethyl,
- oder einen Rest 2-Furyl oder 3-Furyl,
- 2-Thienyl oder 3-Thienyl, oder
- 2-Thiazolyl, 4-Thiazolyl oder 5-Thiazolyl, und
R₄ einen Rest Alkyloxyacetoxy, dessen Teil Alkyl 1 bis 4 Kohlenstoffatome enthält, Cycloalkanoyloxy, dessen Teil Cycloalkanoyl 4 bis 8 Kohlenstoffatome enthält oder Pyridylcarbonyloxy bedeutet und R₅ ein gerader oder verzweigter Rest Alkyloxy mit 1 bis 6 Kohlenstoffatomen ist.

3. Neue Taxoide nach Anspruch 1, worin Z ein Wasserstoffatom oder einen Rest der allgemeinen Formel (II) bedeutet, in der R₁ einen Rest Benzoyl oder einen Rest R₂-O-CO- darstellt, worin R₂ ein Rest tert.-Butyl ist, und R₃ einen Rest Isobutyl, Isobutenyl, Butenyl, Cyclohexyl, Phenyl, 2-Furyl, 3-Furyl, 2-Thienyl, 3-Thienyl, 2-Thiazolyl, 4-Thiazolyl oder 5-Thiazolyl bedeutet, R₄ einen Rest Methoxyacetoxy, Cyclopropylcarbonyloxy, Cyclopentylcarbonyloxy, 2-Pyridyl-carbonyloxy oder 3-Pyridyl-carbonyloxy darstellt und R₅ ein Rest Methoxy ist.

4. Verfahren zur Herstellung der Taxoide nach einem der Ansprüche 1, 2 oder 3, worin Z einen Rest der allgemeinen Formel (II) darstellt, dadurch gekennzeichnet, daß man ein Produkt der allgemeinen Formel (III) 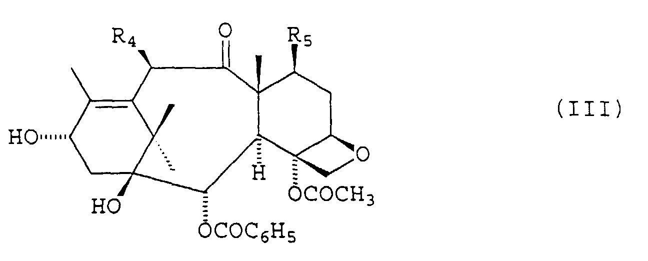 in der R₄ und R₅ wie in einem der Ansprüche 1, 2 oder 3 definiert sind, mit Hilfe einer Säure der allgemeinen Formel (IV) 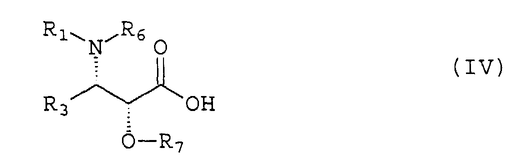 in der R₁ und R₃ wie vorstehend definiert sind, entweder R₆ ein Wasserstoffatom ist und R₇ eine Schutzgruppe für die Hydroxyfunktion darstellt, oder R₆ und R₇ zusammen einen gesättigten Heterocyclus mit 5 oder 6 Ringgliedern bilden, oder mit einem Derivat dieser Säure verestert, um einen Ester der allgemeinen Formel (V) 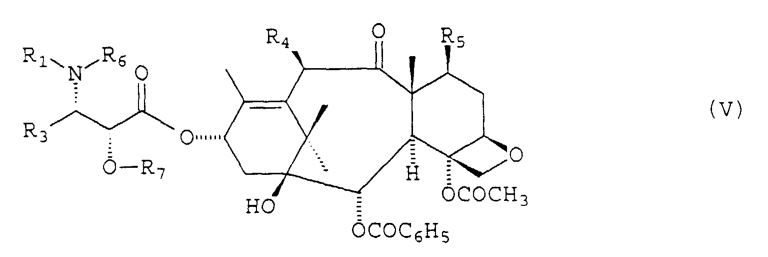 zu erhalten, in der R₁, R₃, R₄, R₅, R₆ und R₇ wie vorstehend definiert sind, bei dem man dann die durch R₇ und/oder R₆ und R₇ dargestellten Schutzgruppen durch Wasserstoffatome ersetzt.

5. Verfahren nach Anspruch 4, dadurch gekennzeichnet, daß die Veresterung mit Hilfe einer Säure der allgemeinen Formel (IV) in Anwesenheit eines Kondensationsmittels und eines Aktivierungsmittels in einem organischen Lösungsmittel bei einer Temperatur zwischen -10 °C und 90 °C durchgeführt wird.

6. Verfahren nach Anspruch 4, dadurch gekennzeichnet, daß die Veresterung mit Hilfe einer Säure der allgemeinen Formel (IV) in Form des symmetrischen Anhydrides durchgeführt wird, wobei man in Anwesenheit eines Aktivierungsmittels in einem organischen Lösungsmittel bei einer Temperatur zwischen 0 °C und 90 °C arbeitet.

7. Verfahren nach Anspruch 4, dadurch gekennzeichnet, daß die Veresterung unter Verwendung der Säure der allgemeinen Formel (IV) in Form von Halogenid oder in Form von gemischtem Anhydrid mit einer aliphatischen oder aromatischen Säure, gegebenenfalls hergestellt in situ, in Anwesenheit einer Base durchgeführt wird, wobei man in einem organischen Lösungsmittel bei einer Temperatur zwischen 0 °C und 80 °C arbeitet.

8. Verfahren nach Anspruch 4, dadurch gekennzeichnet, daß man die Schutzgruppen R₇ und/oder R₆ und R₇ durch Wasserstoffatome ersetzt, indem man je nach ihrer Beschaffenheit in der folgenden Weise verfährt:
1) wenn R₆ ein Wasserstoffatom ist und R₇ eine Schutzgruppe für die Hydroxyfunktion darstellt, so ersetzt man die Schutzgruppen durch Wasserstoffatome mit Hilfe einer Mineralsäure oder organischen Säure, allein verwendet oder in Mischung, wobei man in einem organischen Lösungsmittel, ausgewählt unter den Alkoholen, den Ethern, den Estern, den aliphatischen Kohlenwasserstoffen, den halogenierten aliphatischen Kohlenwasserstoffen, den aromatischen Kohlenwasserstoffen oder den Nitrilen, sowie bei einer Temperatur zwischen -10 °C und 60 °C arbeitet;
2) wenn R₆ und R₇ zusammen einen gesättigten Heterocyclus mit 5 oder 6 Ringgliedern der allgemeinen Formel (VI) 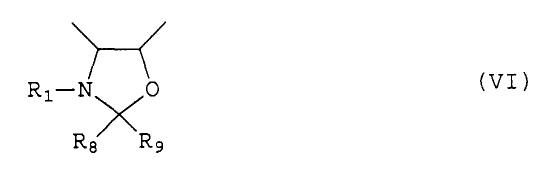 bilden, in der R₁ wie vorstehend definiert ist, R₈ und R₉, gleich oder verschieden, ein Wasserstoffatom oder einen Rest Alkyl mit 1 bis 4 Kohlenstoffatomen, oder einen Rest Aralkyl darstellen, dessen Teil Alkyl 1 bis 4 Kohlenstoffatome enthält und der Teil Aryl vorzugsweise einen Rest Phenyl bedeutet, gegebenenfalls substituiert durch einen oder mehrere Reste Alkoxy mit 1 bis 4 Kohlenstoffatomen, oder einen Rest Aryl darstellen, vorzugsweise einen Rest Phenyl, gegebenenfalls substituiert durch einen oder mehrere Reste Alkoxy mit 1 bis 4 Kohlenstoffatomen, oder auch R₈ einen Rest Alkoxy mit 1 bis 4 Kohlenstoffatomen oder einen Rest Trihalomethyl wie Trichlormethyl, oder einen Rest Phenyl bedeutet, substituiert durch einen Rest Trihalomethyl wie Trichlormethyl, und R₉ ein Wasserstoffatom ist, oder auch R₈ und R₉ zusammen mit dem Kohlenstoffatom, an das sie gebunden sind, einen Zyklus mit 4 bis 7 Ringgliedern bilden, ersetzt man die durch R₆ und R₇ gebildete Schutzgruppe durch Wasserstoffatome, indem man je nach den Bedeutungen von R₁, R₈ und R₉ in der folgenden Weise verfährt:
a) wenn R₁ ein Rest tert.-Butoxycarbonyl ist, R₈ und R₉, gleich oder verschieden, einen Rest Alkyl oder einen Rest Aralkyl oder Aryl darstellen, oder R₈ einen Rest Trihalomethyl oder einen Rest Phenyl bedeutet, substituiert durch einen Rest Trihalomethyl, und R₉ ein Wasserstoffatom ist, oder auch R₈ und R₉ zusammen einen Zyklus mit 4 bis 7 Ringgliedern bilden, so behandelt man den Ester der allgemeinen Formel (V) mit einer Mineralsäure oder organischen Säure, gegebenenfalls in einem organischen Lösungsmittel wie einem Alkohol, um das Produkt der allgemeinen Formel (VII) 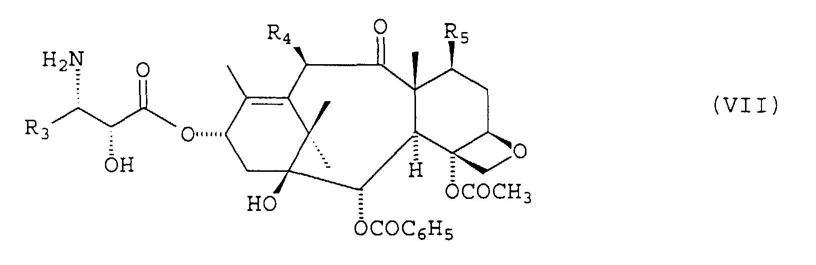 zu erhalten, in der R₃, R₄ und R₅ wie vorstehend definiert sind, das man mit Hilfe von Benzoylchlorid, bei dem der Phenylkern gegebenenfalls substituiert ist, von Thenoylchlorid oder von Furoylchlorid oder mit Hilfe eines Produktes der allgemeinen Formel (VIII)
R₂-O-CO-X (VIII)
acyliert, in der R₂ wie vorstehend definiert ist und X ein Halogenatom oder einen Rest -O-R₂ oder -O-CO-O-R₂ darstellt, um ein Produkt der allgemeinen Formel (I) zu erhalten, in der Z einen Rest der allgemeinen Formel (II) bedeutet,
b) wenn R₁ einen Rest Benzoyl, gegebenenfalls substituiert, Thenoyl oder Furoyl oder einen Rest R₂O-CO- darstellt, worin R₂ wie vorstehend definiert ist, R₈ ein Wasserstoffatom oder einen Rest Alkoxy mit 1 bis 4 Kohlenstoffatomen oder einen Rest Phenyl bedeutet, substituiert durch einen oder mehrere Reste Alkoxy mit 1 bis 4 Kohlenstoffatomen, und R₉ ein Wasserstoffatom ist, so ersetzt man die durch R₆ und R₇ gebildete Schutzgruppe durch Wasserstoffatome in Anwesenheit einer Mineralsäure oder organischen Säure, allein verwendet oder in Mischung sowie in stöchiometrischer oder katalytischer Menge, wobei man in einem organischen Lösungsmittel, ausgewählt unter den Alkoholen, den Ethern, den Estern, den aliphatischen Kohlenwasserstoffen, den halogenierten aliphatischen Kohlenwasserstoffen und den aromatischen Kohlenwasserstoffen, sowie bei einer Temperatur zwischen -10 °C und 60 °C arbeitet.

9. Verfahren zur Herstellung eines neuen Taxoides nach einem der Ansprüche 1, 2 oder 3, worin Z ein Wasserstoffatom ist, R₄ wie in einem der Ansprüche 1, 2 oder 3 definiert ist und R₅ wie in einem der Ansprüche 1, 2 oder 3 definiert ist, dadurch gekennzeichnet, daß man das 10-Desacetyl-Baccatin III der Formel (IX) 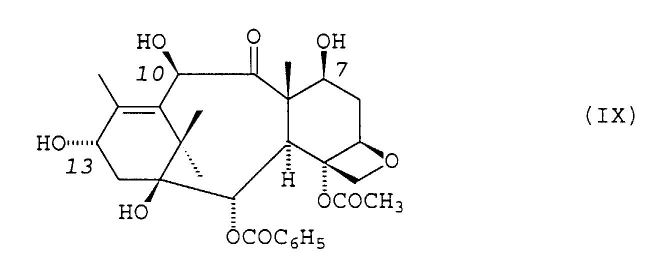 mit einem Silylhalogenid der allgemeinen Formel (X)
(R)₃-Si-Hal (X)
behandelt, in der die Symbole R, gleich oder verschieden, einen Rest Alkyl mit 1 bis 4 Kohlenstoffatomen, gegebenenfalls substituiert durch einen Rest Phenyl, einen Rest Cycloalkyl mit 3 bis 6 Kohlenstoffatomen oder einen Rest Phenyl darstellen, um ein Produkt der allgemeinen Formel (XI) 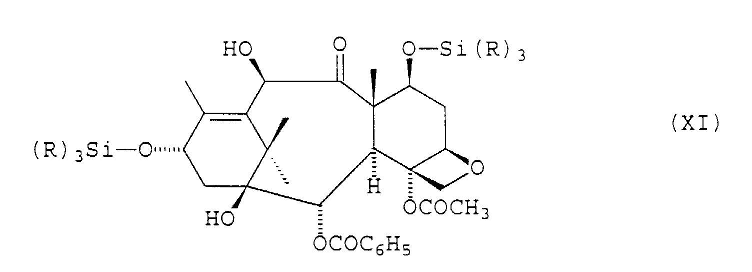 zu erhalten, in der R wie vorstehend definiert ist, das man mit einem Produkt der allgemeinen Formel (XII)
R'₄-X₁ (XII)
behandelt, in der R'₄ wie R'₄-O- ist und identisch mit R₄ wie in einem der Ansprüche 1, 2 oder 3 definiert, und X₁ ein Halogenatom ist, um ein Produkt der allgemeinen Formel (XIII) 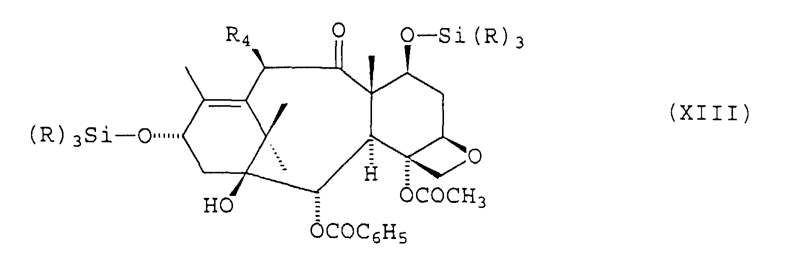 zu erhalten, in der R und R₄ wie vorstehend definiert sind, bei dem man die silylierten Schutzgruppen durch Wasserstoffatome ersetzt, um ein Produkt der allgemeinen Formel (XIV) 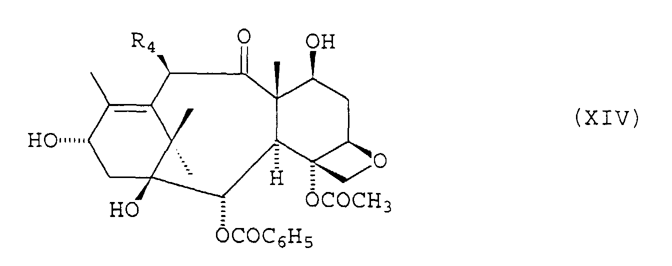 zu erhalten, in der R₄ wie vorstehend definiert ist, das man in Position 7 durch Einwirkung eines Produktes der allgemeinen Formel (XV)
R'₅-X₂ (XV)
selektiv verethert, in der R'₅ wie R'₅-O- ist und identisch mit R₅ wie in einem der Ansprüche 1, 2 oder 3 definiert, und X₂ ein reaktiver Esterrest oder ein Halogenatom ist, um das Produkt der allgemeinen Formel (I) zu ergeben, in der Z ein Wasserstoffatom darstellt.

10. Verfahren zur Herstellung eines Produktes nach einem der Ansprüche 1, 2 oder 3, worin Z einen Rest der allgemeinen Formel (II) darstellt, R₄ wie in einem der Ansprüche 1, 2 oder 3 definiert ist und R₅ wie in einem der Ansprüche 1, 2 oder 3 definiert ist, dadurch gekennzeichnet, daß man ein Produkt der allgemeinen Formel (XVI) 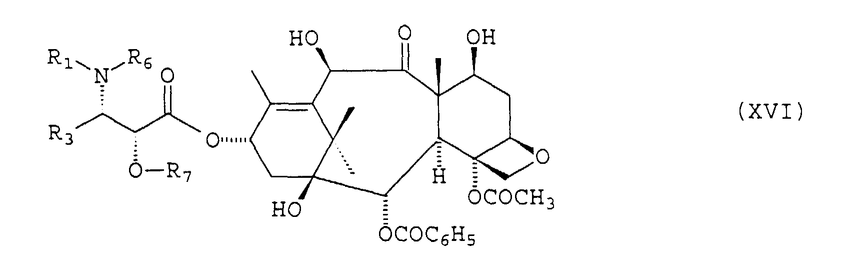 in der R₁, R₃, R₆ und R₇ wie in einem der Ansprüche 1, 2, 3 oder 4 definiert sind, mit einem Produkt der allgemeinen Formel (X)
(R)₃-Si-Hal (X)
behandelt, in der die Symbole R, gleich oder verschieden, einen Rest Alkyl mit 1 bis 4 Kohlenstoffatomen, gegebenenfalls substituiert durch einen Rest Phenyl, einen Rest Cycloalkyl mit 3 bis 6 Kohlenstoffatomen oder einen Rest Phenyl darstellen, um ein Produkt der allgemeinen Formel (XVII) 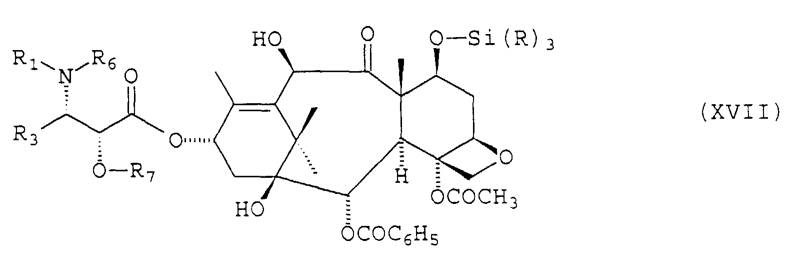 zu erhalten, in der R, R₁, R₃, R₆ und R₇ wie vorstehend definiert sind, das dann in Position 10 mit Hilfe eines Produktes der allgemeinen Formel (XII)
R'₄-X₁ (XII)
funktionalisiert wird, in der R'₄ wie R'₄-O- ist und identisch mit R₄ wie in einem der Ansprüche 1, 2 oder 3 definiert, und X₁ ein Halogenatom oder ein reaktiver Esterrest ist, um ein Produkt der allgemeinen Formel (XVIII) 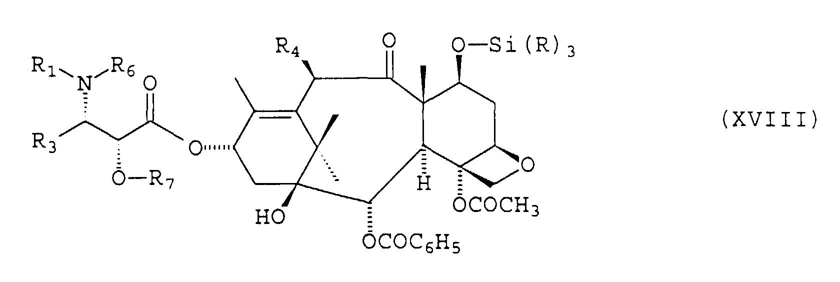 zu ergeben, in der R, R₁, R₃, R₄, R₆ und R₇ wie vorstehend definiert sind, bei dem man dann die silylierte Schutzgruppe durch ein Wasserstoffatom ersetzt, um ein Produkt der allgemeinen Formel (XIX) 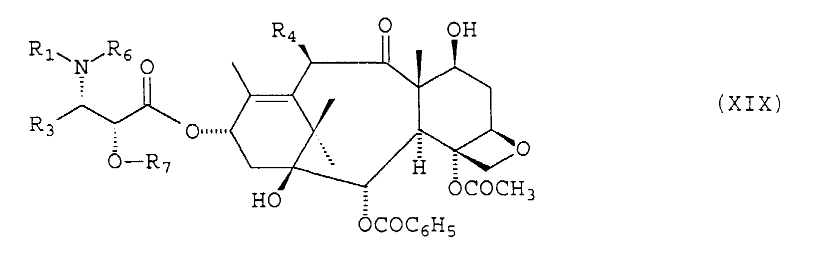 zu ergeben, das durch Einwirkung eines Produktes der allgemeinen Formel (XV) zu einem Produkt der allgemeinen Formel (V) führt, bei dem die Schutzgruppen durch Wasserstoffatome ersetzt werden, um ein Produkt der allgemeinen Formel (I) zu ergeben, in der Z einen Rest der allgemeinen Formel (II) darstellt.

11. Verfahren zur Herstellung eines Produktes nach einem der Ansprüche 1, 2 oder 3, dadurch gekennzeichnet, daß man aktiviertes Raney-Nickel in Anwesenheit eines aliphatischen Alkohols mit 1 bis 3 Kohlenstoffatomen oder eines Ethers mit einem Produkt der allgemeinen Formel (XXI) 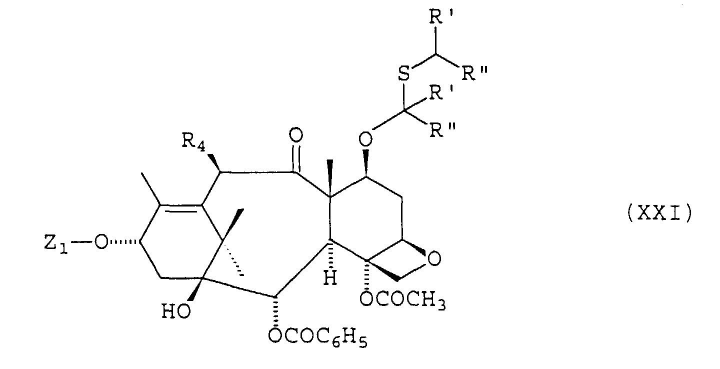 zu Reaktion bringt, in der R₄ wie in einem der Ansprüche 1, 2 oder 3 definiert ist, R' und R", gleich oder verschieden, ein Wasserstoffatom oder einen Rest Alkyl mit 1 bis 6 Kohlenstoffatomen, Alkenyl mit 2 bis 6 Kohlenstoffatomen, Alkinyl mit 3 bis 6 Kohlenstoffatomen, Cycloalkyl mit 2 bis 6 Kohlenstoffatomen oder Cycloalkenyl mit 3 bis 6 Kohlenstoffatomen, gegebenenfalls substituiert, darstellen, oder auch R' und R" zusammen mit dem Kohlenstoffatom, an das sie gebunden sind, einen Rest Cycloalkyl mit 3 bis 6 Kohlenstoffatomen oder einen Rest Cycloalkenyl mit 4 bis 6 Kohlenstoffatomen bilden, und Z₁ ein Wasserstoffatom oder einen Rest der allgemeinen Formel (XXII) 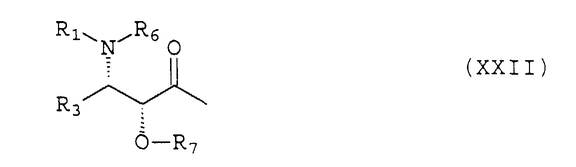 darstellt, in der R₁ und R₃ wie in einem der Ansprüche 1 bis 3 definiert sind und R₆ und R₇ wie in Anspruch 4 definiert sind, um ein Produkt der allgemeinen Formel (XXIII) 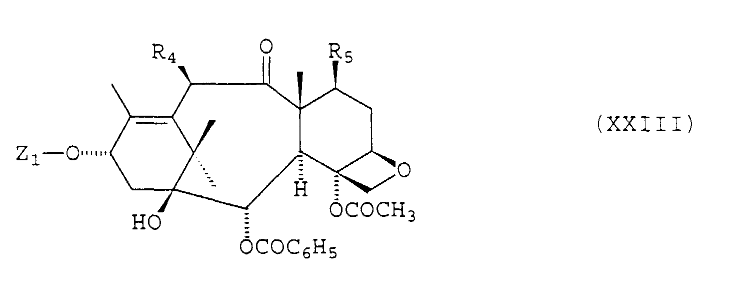 zu erhalten, und man, wenn Z₁ einen Rest der allgemeinen Formel (XXII) darstellt, den Austausch der durch R₆ und/oder R₆ und R₇ dargestellten Schutzgruppen durch Wasserstoffatome unter den Bedingungen von Anspruch 8 anschließt.

12. Verfahren zur Herstellung nach Anspruch 11, dadurch gekennzeichnet, daß man bei einer Temperatur zwischen -10 °C und 60 °C arbeitet.

13. 3-tert.-Butoxycarbonylamino-2-hydroxy-3-phenyl-propionat-(2R,3S) von 4α-Acetoxy-2α-benzoyloxy-1β-hydroxy-5β,20-epoxy-7β-methoxy-10β-methoxyacetoxy-9-oxo-11-taxen-13α-yl.

14. 3-tert.-Butoxycarbonylamino-2-hydroxy-3-phenyl-propionat-(2R,3S) von 4α-Acetoxy-2α-benzoyloxy-1β-hydroxy-5β,20-epoxy-7β-methoxy-10β-cyclopropylcarbonyloxy-9-oxo-11-taxen-13α-yl.

15. 3-tert.-Butoxycarbonylamino-2-hydroxy-3-phenyl-propionat-(2R,3S) von 4α-Acetoxy-2α-benzoyloxy-1β-hydroxy-5β,20-epoxy-7β-methoxy-10β-cyclopentylcarbonyloxy-9-oxo-11-taxen-13α-yl.

16. 3-tert.-Butoxycarbonylamino-2-hydroxy-3-phenyl-propionat-(2R,3S) von 4α-Acetoxy-2α-benzoyloxy-1β-hydroxy-5β,20-epoxy-7β-methoxy-10β-(2-pyridyl)-carbonyloxy-9-oxo-11-taxen-13α-yl.

17. 3-tert.-Butoxycarbonylamino-2-hydroxy-3-phenyl-propionat-(2R,3S) von 4α-Acetoxy-2α-benzoyloxy-1β-hydroxy-5β,20-epoxy-7β-methoxy-10β-(3-pyridyl)-carbonyloxy-9-oxo-11-taxen-13α-yl.

18. Pharmazeutische Zusammensetzung, dadurch gekennzeichnet, daß sie mindestens ein Produkt nach einem der Ansprüche 1, 2 oder 3, worin Z einen Rest der allgemeinen Formel (II) darstellt, in Assoziation mit einem oder mehreren pharmazeutisch akzeptablen Verdünnungsmitteln oder Zusatzstoffen und gegebenenfalls einer oder mehreren kompatiblen und pharmakologisch aktiven Verbindungen enthält.

19. Pharmazeutische Zusammensetzung, dadurch gekennzeichnet, daß sie mindestens das Produkt nach Anspruch 13 in Assoziation mit einem oder mehreren pharmazeutisch akzeptablen Verdünnungsmitteln oder Zusatzstoffen und gegebenenfalls einer oder mehreren kompatiblen und pharmakologisch aktiven Verbindungen enthält.

20. Pharmazeutische Zusammensetzung, dadurch gekennzeichnet, daß sie mindestens das Produkt nach Anspruch 14 in Assoziation mit einem oder mehreren pharmazeutisch akzeptablen Verdünnungsmitteln oder Zusatzstoffen und gegebenenfalls einer oder mehreren kompatiblen und pharmakologisch aktiven Verbindungen enthält.

21. Pharmazeutische Zusammensetzung, dadurch gekennzeichnet, daß sie mindestens das Produkt nach Anspruch 15 in Assoziation mit einem oder mehreren pharmazeutisch akzeptablen Verdünnungsmitteln oder Zusatzstoffen und gegebenenfalls einer oder mehreren kompatiblen und pharmakologisch aktiven Verbindungen enthält.

22. Pharmazeutische Zusammensetzung, dadurch gekennzeichnet, daß sie mindestens das Produkt nach Anspruch 16 in Assoziation mit einem oder mehreren pharmazeutisch akzeptablen Verdünnungsmitteln oder Zusatzstoffen und gegebenenfalls einer oder mehreren kompatiblen und pharmakologisch aktiven Verbindungen enthält.

23. Pharmazeutische Zusammensetzung, dadurch gekennzeichnet, daß sie mindestens das Produkt nach Anspruch 17 in Assoziation mit einem oder mehreren pharmazeutisch akzeptablen Verdünnungsmitteln oder Zusatzstoffen und gegebenenfalls einer oder mehreren kompatiblen und pharmakologisch aktiven Verbindungen enthält.

## Claims

1. New taxoids of general formula: 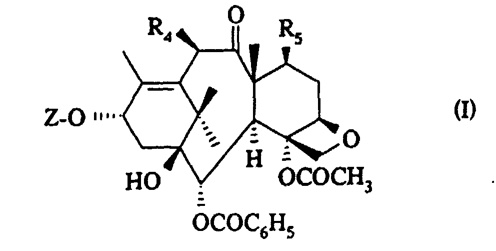 in which:
Z represents a hydrogen atom or a radical of general formula: 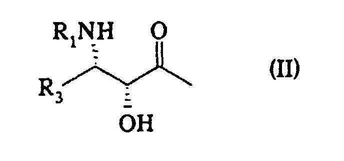 in which:
R₁ represents
- a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals, a thenoyl or furoyl radical
- or a radical R₂-O-CO- in which R₂ represents:
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,
R₃ represents:
- an unbranched or branched alkyl radical containing 1 to 8 carbon atoms,
- an unbranched or branched alkenyl radical containing 2 to 8 carbon atoms,
- an unbranched or branched alkynyl radical containing 2 to 8 carbon atoms,
- a cycloalkyl radical containing 3 to 6 carbon atoms,
- a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
- or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals,
R₄ represents:
- an alkanoyloxy radical in which the alkanoyl portion contains 2 to 6 carbon atoms in an unbranched or branched chain, except for an acetoxy radical,
- an alkenoyloxy radical in which the alkenoyl portion contains 3 to 6 carbon atoms in an unbranched or branched chain,
- an alkynoyloxy radical in which the alkynoyl portion contains 3 to 6 carbon atoms in an unbranched or branched chain,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms, optionally substituted with an alkyl radical containing 1 to 4 carbon atoms or a phenyl radical or a phenylalkyl radical in which the alkyl portion contains 1 to 4 carbon atoms, or alternatively R₄ represents a cycloalkanoyloxy radical in which the cycloalkanoyl portion contains 4 to 8 carbon atoms or a cycloalkenoyloxy radical in which the cycloalkenoyl portion contains 4 to 8 carbon atoms, or alternatively R₄ represents a benzoyloxy or heterocyclylcarbonyloxy radical in which the heterocyclic portion represents a 5- or 6-membered aromatic heterocycle containing one or more hetero atoms chosen from oxygen, sulphur and nitrogen atoms,
R₅ represents:
- an alkoxy radical containing 1 to 6 carbon atoms in an unbranched or branched chain optionally substituted with an alkoxy radical containing 1 to 4 carbon atoms,
- an alkenyloxy radical containing 3 to 6 carbon atoms,
- an alkynyloxy radical containing 3 to 6 carbon atoms,
- a cycloalkyloxy radical containing 3 to 6 carbon atoms
- or a cycloalkenyloxy radical containing 3 to 6 carbon atoms,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms.

2. New taxoids according to claim 1 for which
Z represents a hydrogen atom or a radical of general formula (II) in which R₁ represents a benzoyl radical or a radical R₂-O-CO- in which R₂ represents a tert-butyl radical
and R₃ represents:
- an alkyl radical containing 1 to 6 carbon atoms,
- an alkenyl radical containing 2 to 6 carbon atoms,
- a cycloalkyl radical containing 3 to 6 carbon atoms,
- a phenyl radical
optionally substituted with one or more identical or different atoms or radicals chosen from halogen atoms and alkyl, alkoxy, dialkylamino, acylamino, alkoxycarbonylamino or trifluoromethyl radicals,
- or a 2- or 3-furyl,
- 2- or 3-thienyl or
- 2-, 4- or 5-thiazolyl radical, and
R₄ represents an alkyloxyacetoxy radical in which the alkyl portion contains 1 to 4 carbon atoms, a cycloalkanoyloxy radical in which the cycloalkanoyl portion contains 4 to 8 carbon atoms, or a pyridylcarbonyloxy radical and R₅ represents an unbranched or branched alkyloxy radical containing 1 to 6 carbon atoms.

3. New taxoids according to claim 1 for which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, R₄ represents a methoxyacetoxy, cyclopropylcarbonyloxy, cyclopentylcarbonyloxy, 2-pyridylcarbonyloxy or 3-pyridylcarbonyloxy radical and R₅ represents a methoxy radical.

4. Process for preparing the taxoids according to one of claims 1, 2 and 3 for which Z represents a radical of general formula (II), characterized in that a product of general formula: 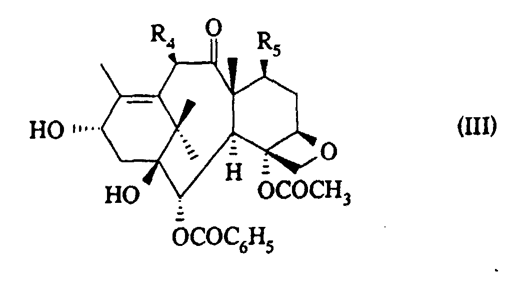 in which R₄ and R₅ are defined as in one of claims 1, 2 and 3, is esterified by means of an acid of general formula: 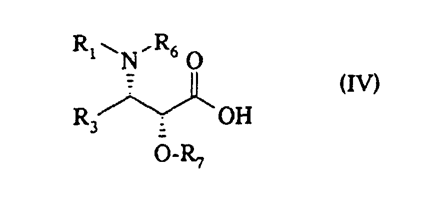 in which R₁ and R₃ are defined as above, and either R₆ represents a hydrogen atom and R₇ represents a group protecting the hydroxyl function, or R₆ and R₇ together form a saturated 5- or 6-membered heterocycle, or by means of a derivative of this acid, to obtain an ester of general formula: 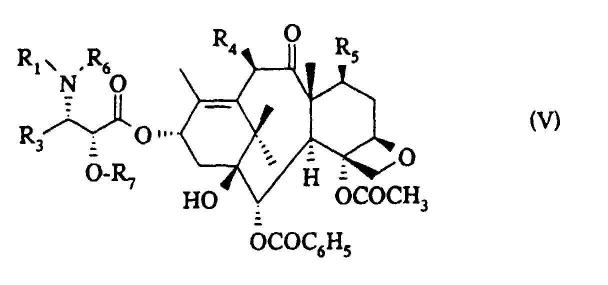 in which R₁, R₃, R₄, R₅, R₆ and R₇ are defined as above, the protective groups of which, represented by R₇ and/or R₆ and R₇, are replaced by hydrogen atoms.

5. Process according to claim 4, characterized in that the esterification is performed by means of an acid of general formula (IV) in the presence of a condensing agent and an activating agent in an organic solvent at a temperature of between -10 and 90°C.

6. Process according to claim 4, characterized in that the esterification is performed by means of an acid of general formula (IV) in the form of the symmetrical anhydride, working in the presence of an activating agent in an organic solvent at a temperature of between 0 and 90°C.

7. Process according to claim 4, characterized in that the esterification is performed using the acid of general formula (IV) in halide form or in the form of a mixed anhydride with an aliphatic or aromatic acid, optionally prepared in situ, in the presence of a base, working in an organic solvent at a temperature of between 0 and 80°C.

8. Process according to claim 4, characterized in that the protective groups R₇ and/or R₆ and R₇ are replaced by hydrogen atoms, working, depending on their nature, in the following manner:
1) when R₆ represents a hydrogen atom and R₇ represents a group protecting the hydroxyl function, the protective groups are replaced by hydrogen atoms by means of an inorganic or organic acid used alone or mixed, working in an organic solvent chosen from alcohols, ethers, esters, aliphatic hydrocarbons, halogenated aliphatic hydrocarbons, aromatic hydrocarbons or nitriles at a temperature of between -10 and 60°C,
2) when R₆ and R₇ together form a saturated 5- or 6-membered heterocycle of general formula: 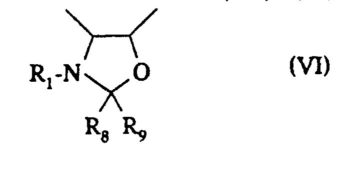 in which R₁ is defined as above and R₈ and R₉, which may be identical or different, represent a hydrogen atom or an alkyl radical containing 1 to 4 carbon atoms, or an aralkyl radical in which the alkyl portion contains 1 to 4 carbon atoms and the aryl portion preferably represents a phenyl radical optionally substituted with one or more alkoxy radicals containing 1 to 4 carbon atoms, or an aryl radical preferably representing a phenyl radical optionally substituted with one or more alkoxy radicals containing 1 to 4 carbon atoms, or alternatively R₈ represents an alkoxy radical containing 1 to 4 carbon atoms or a trihalomethyl radical such as trichloromethyl or a phenyl radical substituted with a trihalomethyl radical such as trichloromethyl and R₉ represents a hydrogen atom, or alternatively R₈ and R₉, together with the carbon atom to which they are linked, form a 4- to 7-membered ring, the protective group formed by R₆ and R₇ is replaced by hydrogen atoms, working, depending on the meanings of R₁, R₈ and R₉, in the following manner:
a) when R₁ represents a tert-butoxycarbonyl radical and R₈ and R₉, which may be identical or different, represent an alkyl radical or an aralkyl or aryl radical, or alternatively R₈ represents a trihalomethyl radical or a phenyl radical substituted with a trihalomethyl radical and R₉ represents a hydrogen atom, or alternatively R₈ and R₉ together form a 4- to 7-membered ring, the ester of general formula (V) is treated with an inorganic or organic acid, where appropriate in an organic solvent such as an alcohol, to obtain the product of general formula: 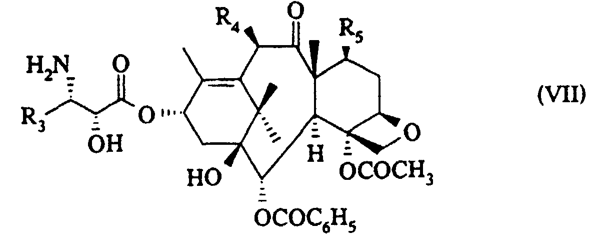 in which R₃, R₄ and R₅ are defined as above, which is acylated by means of benzoyl chloride in which the phenyl ring is optionally substituted or by means of thenoyl chloride, of furoyl chloride or of a product of general formula:
R₂-O-CO-X (VIII)
in which R₂ is defined as above and X represents a halogen atom or a residue -O-R₂ or -O-CO-O-R₂, to obtain a product of general formula (I) in which Z represents a radical of general formula (II),
b) when R₁ represents an optionally substituted benzoyl radical, a thenoyl or furoyl radical or a radical R₂O-CO- in which R₂ is defined as above, R₈ represents a hydrogen atom or an alkoxy radical containing 1 to 4 carbon atoms or a phenyl radical substituted with one or more alkoxy radicals containing 1 to 4 carbon atoms and R₉ represents a hydrogen atom, the protective group formed by R₆ and R₇ is replaced by hydrogen atoms in the presence of an inorganic or organic acid used alone or mixed in a stoichiometric or catalytic amount, and working in an organic solvent chosen from alcohols, ethers, esters, aliphatic hydrocarbons, halogenated aliphatic hydrocarbons and aromatic hydrocarbons at a temperature of between -10 and 60°C.

9. Process for preparing a new taxoid according to one of claims 1, 2 and 3 for which Z represents a hydrogen atom, R₄ is defined as in one of claims 1, 2 and 3 and R₅ is defined as in one of claims 1, 2 and 3, characterized in that 10-deacetylbaccatin III of formula: 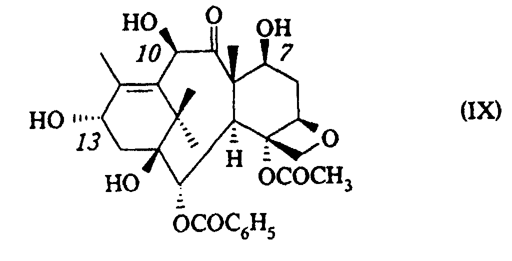 is treated with a silyl halide of general formula:
(R)₃-Si-Hal (X)
in which the symbols R, which may be identical or different, represent an alkyl radical containing 1 to 4 carbon atoms, optionally substituted with a phenyl radical, a cycloalkyl radical containing 3 to 6 carbon atoms or a phenyl radical, to obtain a product of general formula: 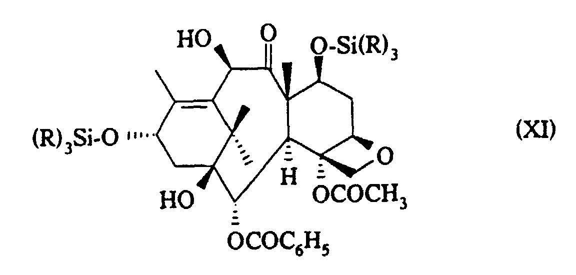 in which R is defined as above, which is treated with a product of general formula:
R'₄-X₁ (XII)
in which R'₄ is such that R'₄-O- is identical to R₄ defined as in one of claims 1, 2 and 3 and X₁ represents a halogen atom, to obtain a product of general formula: 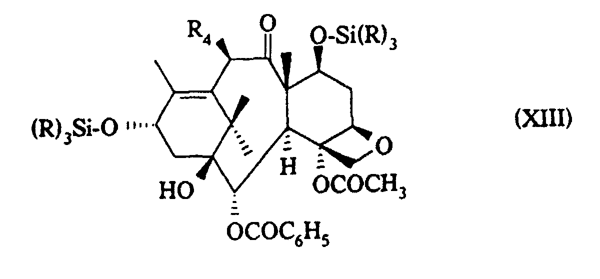 in which R and R₄ are defined as above, the silyl protective groups of which are replaced by hydrogen atoms to obtain a product of general formula: 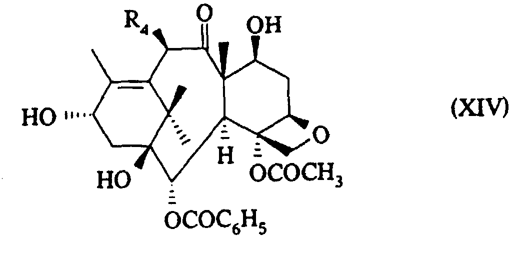 in which R₄ is defined as above, which is etherified selectively at position 7 by the action of a product of general formula:
R'₅-X₂ (XV)
in which R'₅ is such that R'₅-O- is identical to R₅ defined as in one of claims 1, 2 and 3 and X₂ represents a reactive ester residue or a halogen atom, to give the product of general formula (I) in which Z represents a hydrogen atom.

10. Process for preparing a product according to one of claims 1, 2 and 3 for which Z represents a radical of general formula (II), R₄ is defined as in one of claims 1, 2 and 3 and R₅ is defined as in one of claims 1, 2 and 3, characterized in that a product of general formula: 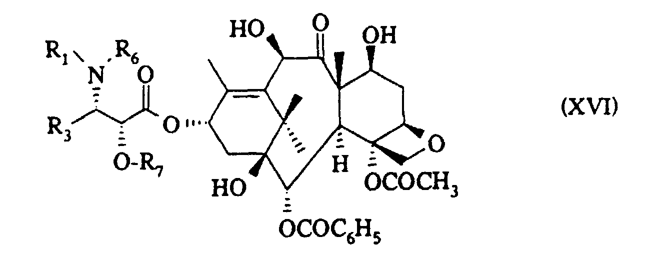 in which R₁, R₃, R₆ and R₇ are defined as in one of claims 1, 2, 3 and 4, is treated by means of a product of general formula:
(R)₃Si-Hal (X)
in which the symbols R, which may be identical or different, represent an alkyl radical containing 1 to 4 carbon atoms, optionally substituted with a phenyl radical, or a cycloalkyl radical containing 3 to 6 carbon atoms or a phenyl radical, to obtain a product of general formula: 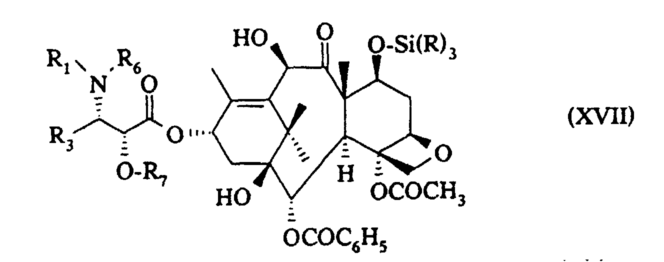 in which R, R₁, R₃, R₆ and R₇ are defined as above, which is functionalized at position 10 by means of a product of general formula:
R'₄-X₁ (XII)
in which R₄ is such that R'₄-O- is identical to R₄ defined as in one of claims 1, 2 and 3 and X₁ represents a halogen atom or a reactive ester residue, to give a product of general formula: 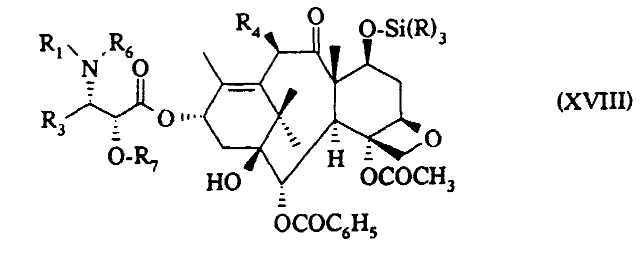 in which R, R₁, R₃, R₄, R₆ and R₇ are defined as above, the silyl protective group of which is replaced by a hydrogen atom to give a product of general formula: 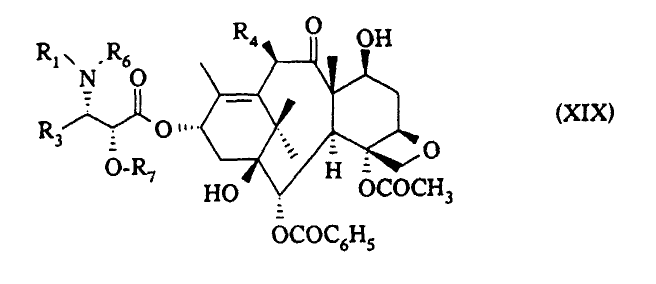 which, by the action of a product of general formula (XV), yields the product of general formula (V), the protective groups of which are replaced by hydrogen atoms to give a product of general formula (I) in which Z represents a radical of general formula (II).

11. Process for preparing a product according to one of claims 1, 2 and 3, characterized in that activated Raney nickel, in the presence of an aliphatic alcohol containing 1 to 3 carbon atoms or an ether, is reacted with a product of general formula: 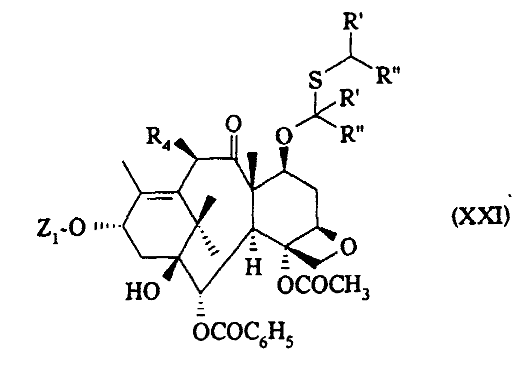 in which R₄ is defined as in one of claims 1, 2 and 3 and R' and R", which may be identical or different, represent a hydrogen atom or an alkyl radical containing 1 to 6 carbon atoms, an alkenyl radical containing 2 to 6 carbon atoms, an alkynyl radical containing 3 to 6 carbon atoms, a cycloalkyl radical containing 2 to 6 carbon atoms or a cycloalkenyl radical containing 3 to 6 carbon atoms, optionally substituted, or alternatively R' and R", together with the carbon atom to which they are linked, form a cycloalkyl radical containing 3 to 6 carbon atoms or a cycloalkenyl radical containing 4 to 6 carbon atoms, and Z₁ represents a hydrogen atom or a radical of general formula: 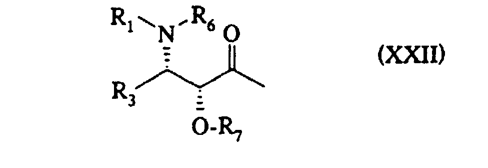 in which R₁ and R₃ are defined as in one of claims 1 to 3 and R₆ and R₇ are defined as in claim 4, to obtain a product of general formula: 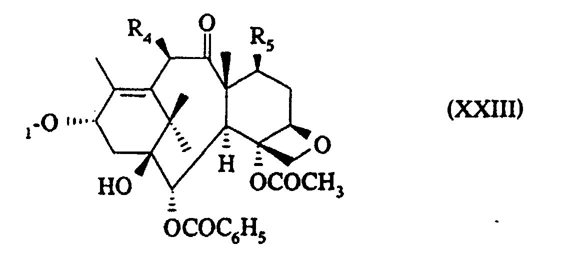 followed, when Z₁ represents a radical of general formula (XXII), by replacement of the protective groups represented by R₆ and/or R₆ and R₇ by hydrogen atoms under the conditions of claim 8.

12. Preparation process according to claim 11, characterized in that it is carried out at a temperature of between -10 and 60°C.

13. 4α-Acetoxy-2α-benzoyloxy-5β,20-epoxy-1β-hydroxy-7β-methoxy-10β-methoxyacetoxy-9-oxo-11-taxen-13α-yl (2R,3S)-3-tert-butoxycarbonylamino-2-hydroxy-3-phenylpropionate.

14. 4α-Acetoxy-2α-benzoyloxy-1β-hydroxy-5β,20-epoxy-7β-methoxy-10β-cyclopropylcarbonyloxy-9-oxo-11-taxen-13α-yl (2R,3S)-3-tert-butoxycarbonylamino-2-hydroxy-3-phenylpropionate.

15. 4α-Acetoxy-2α-benzoyloxy-1β-hydroxy-5β,20-epoxy-7β-methoxy-10β-cyclopentylcarbonyloxy-9-oxo-11-taxen-13α-yl (2R,3S)-3-tert-butoxycarbonylamino-2-hydroxy-3-phenylpropionate.

16. 4α-Acetoxy-2α-benzoyloxy-1β-hydroxy-5β,20-epoxy-7β-methoxy-10β-(2-pyridyl)carbonyloxy-9-oxo-11-taxen-13α-yl (2R,3S)-3-tert-butoxycarbonylamino-2-hydroxy-3-phenylpropionate.

17. 4α-Acetoxy-2α-benzoyloxy-1β-hydroxy-5β,20-epoxy-7β-methoxy-10β-(3-pyridyl)carbonyloxy-9-oxo-11-taxen-13α-yl (2R,3S)-3-tert-butoxycarbonylamino-2-hydroxy-3-phenylpropionate.

18. Pharmaceutical composition, characterized in that it contains at least one product according to one of claims 1, 2 and 3 for which Z represents a radical of general formula (II), in combination with one or more pharmaceutically acceptable diluents or adjuvants and optionally one or more compatible and pharmacologically active compounds.

19. Pharmaceutical composition, characterized in that it contains at least the product according to claim 13 in combination with one or more pharmaceutically acceptable diluents or adjuvants and optionally one or more compatible and pharmacologically active compounds.

20. Pharmaceutical composition, characterized in that it contains at least the product according to claim 14 in combination with one or more pharmaceutically acceptable diluents or adjuvants and optionally one or more compatible and pharmacologically active compounds.

21. Pharmaceutical composition, characterized in that it contains at least the product according to claim 15 in combination with one or more pharmaceutically acceptable diluents or adjuvants and optionally one or more compatible and pharmacologically active compounds.

22. Pharmaceutical composition, characterized in that it contains at least the product according to claim 16 in combination with one or more pharmaceutically acceptable diluents or adjuvants and optionally one or more compatible and pharmacologically active compounds.

23. Pharmaceutical composition, characterized in that it contains at least the product according to claim 17 in combination with one or more pharmaceutically acceptable diluents or adjuvants and optionally one or more compatible and pharmacologically active compounds.
